# EUROPEAN PATENT APPLICATION

(11) **EP 4 477 753 A1**
(43) Date of publication of application: **18.12.2024**
(21) Application number: 23752966.4
(22) Date of filing: 10.02.2023
(51) Int. Cl.: C12N 15/63, A61K 31/495, A61K 31/711, A61K 31/713, A61K 45/00, A61K 47/18, A61K 47/22, A61K 48/00, C07C 211/27, C07C 217/22, C07C 229/38, C07D 295/135, C07D 295/155

(54) **NUCLEIC ACID DELIVERY MATERIAL**

(30) Priority: 10.02.2022 JP 2022019637
(71) Applicant: National University Corporation Tokai National Higher Education and Research System, Nagoya-shi, Aichi 464-8601 (JP)
(72) Inventor: ITAMI, Kenichiro, Nagoya-shi, Aichi 464-8601 (JP); AMAIKE, Kazuma, Nagoya-shi, Aichi 464-8601 (JP); KATO, Erika, Nagoya-shi, Aichi 464-8601 (JP); SATO, Ayato, Nagoya-shi, Aichi 464-8601 (JP); NAKAMURA, Masayoshi, Nagoya-shi, Aichi 464-8601 (JP)
(74) Representative: Hoffmann Eitle
(86) International application number: PCT/JP2023/004642
(87) International publication number: WO 2023/153504

(57) **Abstract**

A nucleic acid delivery material comprising a polycyclic aromatic compound having one or more primary, secondary, or tertiary amino groups or nitrogen cation-containing groups and having no perylene diimide ring has excellent nucleic acid delivery efficiency while being a small molecule that can be precisely synthesized.

## Description

### Technical Field

The present invention relates to a nucleic acid delivery material.

### Background Art

Nucleic acid delivery is a method for introducing DNA, RNA, etc. from the outside into host cells, and is an absolutely essential technology for life science research, from the site of drug discovery and breeding to basic biology. Conventional nucleic acid delivery methods include, for example, the lipofection method using a compound, the microinjection method for physical introduction, the particle gun method, and the Agrobacterium method using microorganisms. Now that it is necessary to further advance the understanding and control of life phenomena, various functions, such as nucleic acid delivery efficiency, tissue selectivity, and bioorthogonality, are required for nucleic acid delivery. This is why the emergence of completely new methods and a group of carrier molecules is eagerly awaited in the field of nucleic acid delivery. However, it was necessary to select a different method depending on the structure, size, etc. of the target.

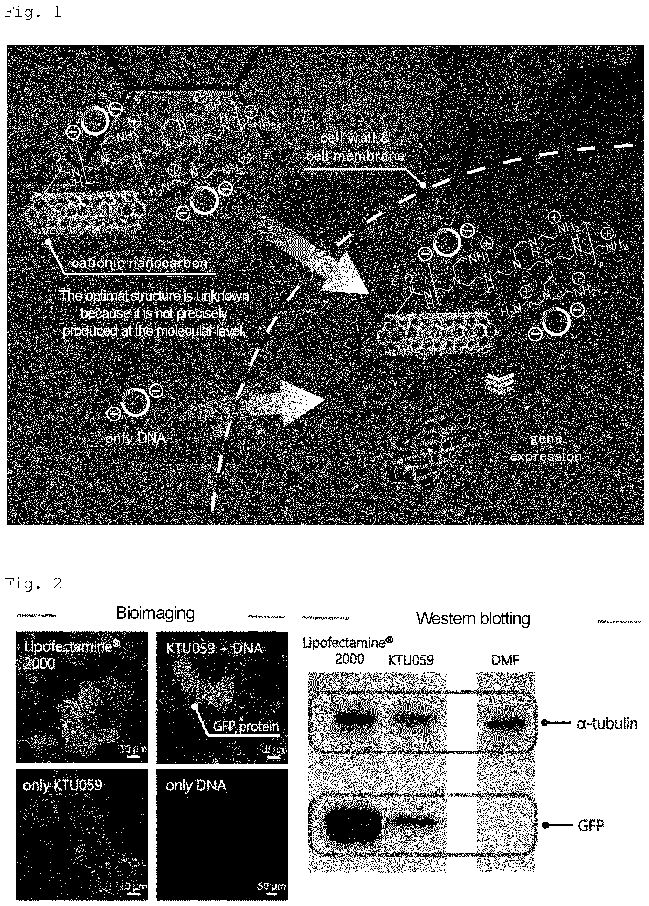

Nanometer-sized carbon materials (nanocarbons), such as carbon nanotubes and graphene, are expected to be next-generation gene delivery carrier molecules due to their high biocompatibility (see, for example, NPL 1). These nanocarbons are usually cationic, and they are complexed with anionic nucleic acids using electrical interactions and introduced into cells (Fig. 1). In recent years, cationic carbon nanotubes have made it possible to deliver nucleic acids to plant cells, mouse kidneys, and the like, and are expected to be used in applied research for genome editing technology, nucleic acid medicine, etc. (see, for example, NPL 2). However, nanocarbons, which have so far been used as nucleic acid delivery materials, are complex mixtures that do not have a single, defined structure. Therefore, the correlation between nanocarbon structure and nucleic acid delivery remains unclear. Although attempts to precisely synthesize such nanocarbons and conduct structural correlation studies have been made using cationic fullerenes, which are 0-dimensional (spherical) nanocarbons (see, for example, NPL 4), carbon nanotubes with a one-dimensional structure, graphene with a two-dimensional structure, and the like that are most often used remain untouched.

### Citation List

### Non-patent Literature

NPL 1: Chem. Rev. 2019, 119, 9559.
NPL 2: Nat. Nanotechnol. 2019, 14, 456
NPL 3: Sci. Transl. Med. 2016, 8, 331ra39.
NPL 4: Chem. Asian. J. 2006, 1, 167.

### Summary of Invention

### Technical Problem

The present invention is intended to solve the above problems, and an object thereof is to provide a nucleic acid delivery material having excellent nucleic acid delivery efficiency by using small molecules that can be precisely synthesized.

### Solution to Problem

As a result of extensive research in view of the above object, the present inventors found that polycyclic aromatic compounds having one or more primary, secondary or tertiary amino groups or nitrogen cation-containing groups have excellent nucleic acid delivery efficiency while being small molecules that can be precisely synthesized. That is, the present invention includes the following configurations.

Item 1. A nucleic acid delivery material comprising a polycyclic aromatic compound having one or more primary, secondary, or tertiary amino groups or nitrogen cation-containing groups and having no perylene diimide ring.

Item 2. The nucleic acid delivery material according to Item 1, wherein the polycyclic aromatic compound is a compound represented by formula (1): 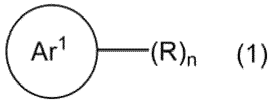 wherein Ar¹ represents an optionally substituted fused aromatic ring other than a perylene diimide ring, R represents a group having a primary, secondary, or tertiary amino group or nitrogen cation-containing group, n represents an integer of 1 or more, and when n is an integer of 2 or more, n Rs may be the same or different.

Item 3. The nucleic acid delivery material according to Item 1 or 2, wherein the polycyclic aromatic compound is a compound represented by formula (1A): 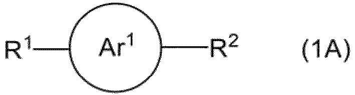 wherein Ar¹ represents an optionally substituted fused aromatic ring other than a perylene diimide ring, and R¹ and R² are the same or different and each represents a group having a primary, secondary, or tertiary amino group or nitrogen cation-containing group.

Item 4. The nucleic acid delivery material according to any one of Items 1 to 3, wherein Ar¹ is a fused aromatic hydrocarbon ring.

Item 5. The nucleic acid delivery material according to any one of Items 2 to 4, wherein the group having a primary, secondary, or tertiary amino group or nitrogen cation-containing group is a group represented by formula (2A) or (2B): 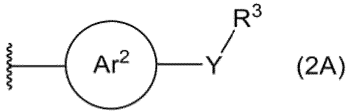 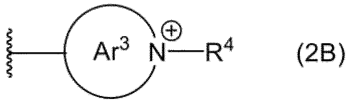 wherein Ar² represents an optionally substituted aromatic ring other than a perylene diimide ring, Ar³ represents an optionally substituted nitrogen-containing heterocyclic ring other than a perylene diimide ring, R³ represents a primary, secondary, or tertiary amino group or nitrogen cation-containing group, R⁴ represents a hydrogen atom or a hydrocarbon group, and Y represents a divalent group.

Item 6. The nucleic acid delivery material according to any one of Items 1 to 5, wherein the primary, secondary, or tertiary amino group or nitrogen cation-containing group is a primary amino group, an optionally substituted imidazolium group, an optionally substituted pyridinium group, an optionally substituted tetraalkylammonium group, or an optionally substituted triazolium group.

Item 7. The nucleic acid delivery material according to any one of Items 2 to 6, wherein the fused aromatic ring has 2 to 10 rings.

Item 8. The nucleic acid delivery material according to any one of Items 1 to 7, further comprising a physiological buffer with a pH of 6.0 to 8.0.

Item 9. The nucleic acid delivery material according to any one of Items 1 to 8, which is a nucleic acid delivery material for at least one member selected from the group consisting of mammalian cells, plant cells, and intestinal bacteria.

Item 10. The nucleic acid delivery material according to any one of Items 1 to 9, wherein the nucleic acid is plasmid DNA.

Item 11. A pharmaceutical composition comprising the nucleic acid delivery material according to any one of Items 1 to 10 and a nucleic acid.

Item 12. A gene therapy agent comprising the pharmaceutical composition according to Item 11.

Item 13. A genome editing composition comprising the nucleic acid delivery material according to any one of Items 1 to 10 and a nucleic acid.

Item 14. A compound represented by formula (1'): 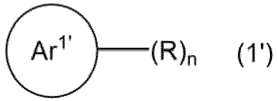 wherein Ar¹' represents an optionally substituted fused aromatic ring having 4 or more rings other than a pyrene ring and a perylene diimide ring, R represents a group having a primary, secondary, or tertiary amino group or nitrogen cation-containing group, n represents an integer of 1 or more, and when n is an integer of 2 or more, n Rs may be the same or different, provided that when Ar¹' is a perylene ring, n Rs bind to n-number of positions 1, 2, 5, 6, 7, 8, 11, and 12 in Ar¹'.

Item 15. The compound according to Item 14, which is represented by formula (1A'): 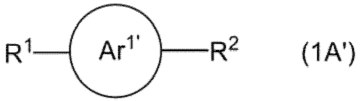 wherein Ar¹' represents an optionally substituted fused aromatic ring having 4 or more rings other than a pyrene ring and a perylene diimide ring, and R¹ and R² are the same or different and each represents a group having a primary, secondary, or tertiary amino group or nitrogen cation-containing group, provided that when Ar¹ is a perylene ring, R¹ and R² bind to two of positions 1, 2, 5, 6, 7, 8, 11, and 12 in Ar¹.

Item 16. The compound according to Item 14 or 15, wherein Ar¹ is a fused aromatic hydrocarbon ring having 5 or more rings.

Item 17. The compound according to any one of Items 14 to 16, which is represented by formula (1A1) or (1A2): 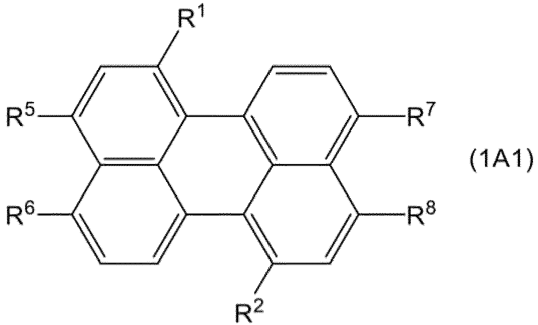 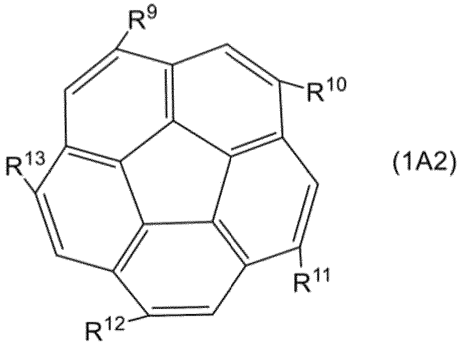 wherein R¹, R², R⁹, R¹⁰, R¹¹, R¹², and R¹³ are the same or different and each represents a group having a primary, secondary, or tertiary amino group or nitrogen cation-containing group, and R³, R⁴, R⁵, and R⁶ are the same or different and each represents a hydrogen atom, a hydroxyl group, a halogen atom, an alkyl group, an alkoxy group, an aryl group, or an alkoxycarbonyl group, provided that at least one of R³, R⁴, R⁵, and R⁶ represents a hydroxyl group, an alkoxy group, or an alkoxycarbonyl group.

Item 18. The compound according to any one of Items 14 to 17, wherein the group having a primary, secondary, or tertiary amino group or nitrogen cation-containing group is a group represented by formula (2A) or (2B):

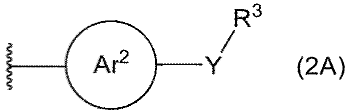

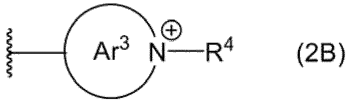

wherein Ar² represents an optionally substituted aromatic ring, Ar³ represents an optionally substituted nitrogen-containing heterocyclic ring, R³ represents a primary, secondary, or tertiary amino group or nitrogen cation-containing group, R⁴ represents a hydrogen atom or a hydrocarbon group, and Y represents a divalent group.

### Advantageous Effects of Invention

According to the present invention, it is possible to provide a nucleic acid delivery material having excellent nucleic acid delivery efficiency by using small molecules that can be precisely synthesized.

### Brief Description of Drawings

Fig. 1 is a schematic diagram showing nucleic acid delivery by nanocarbon.
Fig. 2 shows the results of bioimaging and Western blotting analyses in Test Example 3.
Fig. 3 shows the results of cell viability measurement by trypan blue staining in Test Example 3.
Fig. 4 shows the results of a delivery test to *E. coli* in Test Example 4.
Fig. 5 shows the results of a delivery test to plant cells in Test Example 5.

### Description of Embodiments

In the present specification, the term "comprise" or "contain" is a concept that encompasses both of the meanings of "consist essentially of" and "consist of."

Further, in the present specification, the numerical range indicated by "A to B" means A or more and B or less.

### 1. Nucleic Acid Delivery Material

The nucleic acid delivery material of the present invention comprises a polycyclic aromatic compound having one or more primary, secondary or tertiary amino groups or nitrogen cation-containing groups and having no perylene diimide ring.

### (1-1) Polycyclic Aromatic Compound

The polycyclic aromatic compound refers to a compound composed of an optionally substituted fused aromatic ring other than a perylene diimide ring. That is, the polycyclic aromatic compound used for the nucleic acid delivery material of the present invention is preferably a compound represented by formula (1) : 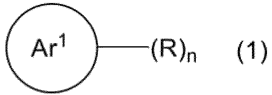 wherein Ar¹ represents an optionally substituted fused aromatic ring other than a perylene diimide ring, R represents a group having a primary, secondary, or tertiary amino group or nitrogen cation-containing group, n represents an integer of 1 or more, and when n is an integer of 2 or more, n Rs may be the same or different.

In formula (1), n represents an integer of 1 or more. n can vary depending on the number of aromatic rings in Ar¹; however, in terms of ease of precision synthesis and ease of improving nucleic acid delivery efficiency, n is preferably an integer of 1 to 5, more preferably an integer of 2 to 4, even more preferably 2 or 3, and particularly preferably 2.

Therefore, the polycyclic aromatic compound used for the nucleic acid delivery material of the present invention is preferably a compound represented by formula (1A): 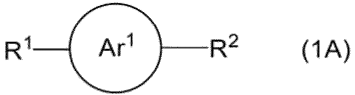 wherein Ar¹ represents an optionally substituted fused aromatic ring other than a perylene diimide ring, and R¹ and R² are the same or different and each represents a group having a primary, secondary, or tertiary amino group or nitrogen cation-containing group.

The number of rings in the fused aromatic ring constituting the polycyclic aromatic compound used for the nucleic acid delivery material of the present invention, i.e., the fused aromatic ring represented by Ar¹ in formulas (1) and (1A), is not particularly limited, but is preferably 2 to 10, more preferably 3 to 8, and even more preferably 4 to 6, in terms of ease of precision synthesis and ease of improving nucleic acid delivery efficiency.

The fused aromatic ring constituting the polycyclic aromatic compound used for the nucleic acid delivery material of the present invention is not particularly limited as long as it is a fused aromatic ring other than a perylene diimide ring. Any fused heteroaromatic ring other than a fused aromatic hydrocarbon ring and a perylene diimide ring can be used; however, in terms of ease of precision synthesis and ease of improving nucleic acid delivery efficiency, a fused aromatic hydrocarbon ring is preferred. Specific examples of such a fused aromatic ring include a naphthalene ring, an anthracene ring, a phenanthrene ring, a tetracene ring, a pyrene ring, a chrysene ring, a triphenylene ring, a pentacene ring, a perylene ring, a benzopyrene ring, a coronene ring, a corannulene ring, and the like.

The fused aromatic ring constituting the polycyclic aromatic compound used for the nucleic acid delivery material of the present invention, that is, the fused aromatic ring represented by Ar¹ in formulas (1) and (1A), may be substituted. Examples of substituents include, but are not particularly limited to, hydroxyl groups, halogen atoms (e.g., fluorine, chlorine, bromine, and iodine atoms), alkyl groups (e.g., methyl, ethyl, and n-propyl groups), alkoxy groups (e.g., methoxy, ethoxy, and n-propoxy groups), the aryl groups mentioned above, alkoxycarbonyl groups (e.g., methoxycarbonyl, ethoxycarbonyl, n-propoxycarbonyl, and n-butyloxycarbonyl groups), and the like.

Taking into consideration the fact that the nucleic acid delivery material of the present invention is used to deliver nucleic acids, i.e., used inside the body, it is preferable to employ substituents that are likely to improve solubility in physiological buffers, i.e., that are likely to improve water solubility. From such a viewpoint, the substituents in the fused aromatic ring presented by Ar¹ in formulas (1) and (1A) are preferably hydroxyl groups, alkoxy groups (e.g., methoxy, ethoxy, and n-propoxy groups), alkoxycarbonyl groups (e.g., methoxycarbonyl, ethoxycarbonyl, n-propoxycarbonyl, and n-butyloxycarbonyl groups), and the like; more preferably hydroxyl groups, alkoxycarbonyl groups (e.g., methoxycarbonyl, ethoxycarbonyl, n-propoxycarbonyl, and n-butyloxycarbonyl groups), and the like; and even more preferably alkoxycarbonyl groups (e.g., methoxycarbonyl, ethoxycarbonyl, n-propoxycarbonyl, and n-butyloxycarbonyl groups), and the like.

The number of such substituents, when present, is not particularly limited, but is preferably 1 to 10, more preferably 2 to 8, and even more preferably 3 to 6, in terms of ease of precision synthesis and ease of improving water solubility.

The nucleic acid delivery material having such a fused aromatic ring of the present invention is preferably a compound represented by formula (1A1) or (1A2): 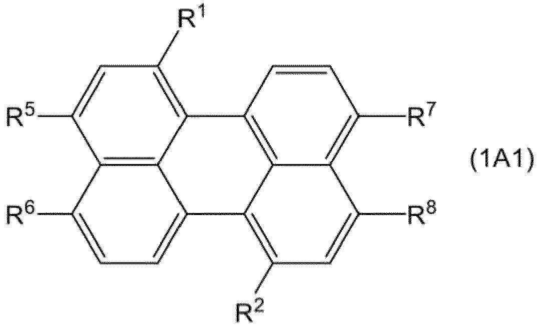 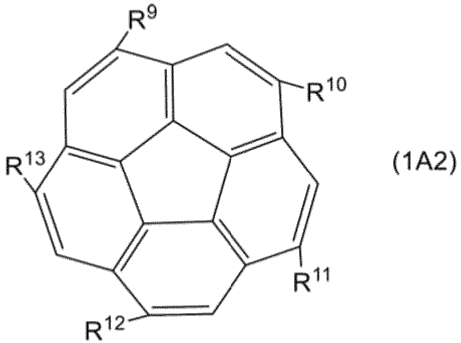 wherein R¹, R², R⁹, R¹⁰, R¹¹, R¹², and R¹³ are the same or different and each represents a group having a primary, secondary, or tertiary amino group or nitrogen cation-containing group, and R³, R⁴, R⁵, and R⁶ are the same or different and each represents a hydrogen atom, a hydroxyl group, a halogen atom, an alkyl group, an alkoxy group, an aryl group, or an alkoxycarbonyl group, provided that at least one of R³, R⁴, R⁵, and R⁶ represents a hydroxyl group, an alkoxy group, or an alkoxycarbonyl group.

In the compound represented by formula (1A1) (perylene compound), the substituents in the fused aromatic ring, that is, R³, R⁴, R⁵, and R⁶ in formula (1A1), represent hydrogen atoms, hydroxyl group, halogen atoms (e.g., fluorine, chlorine, bromine, and iodine atoms), alkyl groups (e.g., methyl, ethyl, and n-propyl groups), alkoxy groups (e.g., methoxy, ethoxy, and n-propoxy groups), the aryl groups mentioned above, or alkoxycarbonyl groups (e.g., methoxycarbonyl, ethoxycarbonyl, n-propoxycarbonyl, and n-butyloxycarbonyl groups).

Taking into consideration the fact that the nucleic acid delivery material of the present invention is used to deliver nucleic acids, i.e., used inside the body, it is preferable to employ substituents that are likely to improve solubility in physiological buffers, i.e., that are likely to improve water solubility. From such a viewpoint, R³, R⁴, R⁵, and R⁶ in formula (1A1) are preferably hydroxyl groups, alkoxy groups (e.g., methoxy, ethoxy, and n-propoxy groups), alkoxycarbonyl groups (e.g., methoxycarbonyl, ethoxycarbonyl, n-propoxycarbonyl, and n-butyloxycarbonyl groups), and the like; more preferably hydroxyl groups, alkoxycarbonyl groups (e.g., methoxycarbonyl, ethoxycarbonyl, n-propoxycarbonyl, and n-butyloxycarbonyl groups), and the like; and even more preferably alkoxycarbonyl groups (e.g., methoxycarbonyl, ethoxycarbonyl, n-propoxycarbonyl, and n-butyloxycarbonyl groups), and the like.

Similarly, in terms of ease of precision synthesis and ease of improving water solubility, in the compound represented by formula (1A1) (perylene compound), 1 to 4, preferably 2 to 4, and more preferably 3 or 4, of R³, R⁴, R⁵, and R⁶ are preferably hydroxyl groups, alkoxy groups, alkoxycarbonyl groups, or the like; more preferably hydroxyl groups, alkoxycarbonyl groups, or the like; and even more preferably alkoxycarbonyl groups or the like.

The group having a primary, secondary, or tertiary amino group or nitrogen cation-containing group contained in the nucleic acid delivery material of the present invention is not particularly limited, but is preferably, in terms of ease of precision synthesis and ease of improving water solubility, a group represented by formula (2A) or (2B): 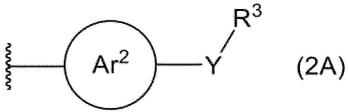 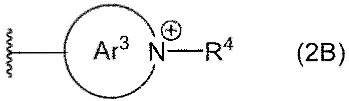 wherein Ar² represents an optionally substituted aromatic ring other than a perylene diimide ring, Ar³ represents an optionally substituted nitrogen-containing heterocyclic ring other than a perylene diimide ring, R³ represents a primary, secondary, or tertiary amino group or nitrogen cation-containing group, R⁴ represents a hydrogen atom or a hydrocarbon group, and Y represents a divalent group.

In the group having a primary, secondary, or tertiary amino group or nitrogen cation-containing group (R¹ and R² in formulas (1), (1A), and (1A1), or R⁹, R¹⁰, R¹¹, R¹², and R¹³ in formula (1A2)), or the primary, secondary, or tertiary amino group or nitrogen cation-containing group (R³ and R⁴ in formulas (2A) and (2B)), contained in the nucleic acid delivery material of the present invention, examples of the primary, secondary, or tertiary amino group include, in addition to amino groups (unsubstituted amino groups), which are primary amino groups, Cₗ₋₆ (preferably C₁₋₄) alkylamino groups, such as N-methylamino, N-ethylamino, N-n-propylamino, and N-isopropylamino groups; C₂₋₁₀ (preferably C₂₋₈) dialkylamino groups, such as dimethylamino, diethylamino, di(n-propyl)amino, and diisopropylamino groups; and cyclic amino groups, such as a piperazino group. In terms of ease of precision synthesis and ease of improving water solubility, preferred are among these are amino groups (unsubstituted amino groups), which are primary amino groups, an N-methylamino group, a piperazino group, and the like; and more preferred are amino groups (unsubstituted amino groups), which are primary amino groups, a piperazino group, and the like.

In the group having a primary, secondary or tertiary amino group or nitrogen cation-containing group (R¹ and R² in formulas (1), (1A), and (1A1), or R⁹, R¹⁰, R¹¹, R¹², and R¹³ in formula (1A2)), or the primary, secondary or tertiary amino group or nitrogen cation-containing group (R³ and R⁴ in formulas (2A) and (2B)), contained in the nucleic acid delivery material of the present invention, examples of the nitrogen cation-containing group include an imidazolium group, a pyridinium group, a tetraalkylammonium group, a triazolium group, and the like. The alkyl groups in the tetraalkylammonium group may be the same or different, and usable examples include C₁₋₆ (preferably C₁₋₄) alkyl groups, such as methyl, ethyl, and n-propyl groups.

The nitrogen cation-containing group may be substituted. Examples of substituents that the nitrogen cation-containing group may have include hydroxyl groups, alkoxy groups (e.g., methoxy, ethoxy, and n-propoxy groups), alkoxycarbonyl groups (e.g., methoxycarbonyl, ethoxycarbonyl, n-propoxycarbonyl, and n-butyloxycarbonyl groups), and the like.

The number of such substituents, when present, is not particularly limited, and can be, for example, 1 to 6, preferably 1 to 4, and more preferably 1 or 2.

Taking into consideration the fact that the nucleic acid delivery material of the present invention is used inside the body, it is preferable that the toxicity of the nucleic acid delivery material of the present invention is as low as possible. For this reason, the group having a primary, secondary, or tertiary amino group or nitrogen cation-containing group (R¹ and R² in formulas (1), (1A), and (1A1), or R⁹, R¹⁰, R¹¹, R¹², and R¹³ in formula (1A2)), and the primary, secondary, or tertiary amino group or nitrogen cation-containing group (R³ in formula (2A)) are preferably primary, secondary, or tertiary amino groups. However, even when a nitrogen cation-containing group is used, it is possible to reduce toxicity by, for example, diluting the concentration.

In formula (2A), the aromatic ring represented by Ar² other than a perylene diimide ring is not particularly limited as long as it is an aromatic ring other than a perylene diimide ring. One having about 1 to 10 rings, and preferably about 1 to 8 rings, can be used, and any heteroaromatic ring other than an aromatic hydrocarbon ring and a perylene diimide ring can be used. However, in terms of ease of precision synthesis and ease of improving nucleic acid delivery efficiency, preferred is a fused aromatic hydrocarbon ring. Specific examples of such aromatic rings include a benzene ring, a naphthalene ring, an anthracene ring, a phenanthrene ring, a tetracene ring, a pyrene ring, a chrysene ring, a triphenylene ring, a pentacene ring, a perylene ring, a benzopyrene ring, a coronene ring, and the like.

The aromatic ring represented by Ar² in formula (2A) may be substituted. Examples of substituents include, but are not particularly limited to, hydroxyl groups, halogen atoms (e.g., fluorine, chlorine, bromine, and iodine atoms), alkyl groups (e.g., methyl, ethyl, and n-propyl groups), alkoxy groups (e.g., methoxy, ethoxy, and n-propoxy groups), the aryl groups mentioned above, alkoxycarbonyl groups (e.g., methoxycarbonyl, ethoxycarbonyl, n-propoxycarbonyl, and n-butyloxycarbonyl groups), and the like.

Taking into consideration the fact that the nucleic acid delivery material of the present invention is used to deliver nucleic acids, i.e., used inside the body, it is preferable to employ substituents that are likely to improve solubility in physiological buffers, i.e., that are likely to improve water solubility. From such a viewpoint, the substituents in the aromatic ring represented by Ar² in formula (2A) are preferably hydroxyl groups, alkoxy groups (e.g., methoxy, ethoxy, and n-propoxy groups), alkoxycarbonyl groups (e.g., methoxycarbonyl, ethoxycarbonyl, n-propoxycarbonyl, and n-butyloxycarbonyl groups), and the like; more preferably hydroxyl groups, alkoxycarbonyl groups (e.g., methoxycarbonyl, ethoxycarbonyl, n-propoxycarbonyl, and n-butyloxycarbonyl groups), and the like; and even more preferably alkoxycarbonyl groups (e.g., methoxycarbonyl, ethoxycarbonyl, n-propoxycarbonyl, and n-butyloxycarbonyl groups), and the like.

The number of such substituents, when present, is not particularly limited, but is preferably 1 to 10, more preferably 2 to 8, and even more preferably 3 to 6, in terms of ease of precision synthesis and ease of improving water solubility.

In formula (2B), the nitrogen-containing heterocyclic ring represented by Ar³ other than a perylene diimide ring is not particularly limited as long as it is a nitrogen-containing heterocyclic ring other than a perylene diimide ring, and one having about 1 to 10 rings, and preferably about 1 to 8 rings, can be used. Specific examples include a pyrrole ring, a pyridine ring, an imidazole ring, a pyrazole ring, an oxazole ring, a thiazole ring, a pyridazine ring, a pyrimidine ring, a pyrazine ring, an indole ring, an isoindole ring, a benzimidazole ring, a quinoline ring, an isoquinoline ring, a quinoxaline ring, a carbazole ring, and the like.

The nitrogen-containing heterocyclic ring represented by Ar³ in formula (2B) may be substituted. Examples of substituents include, but are not particularly limited to, hydroxyl groups, halogen atoms (e.g., fluorine, chlorine, bromine, and iodine atoms), alkyl groups (e.g., methyl, ethyl, and n-propyl groups), alkoxy groups (e.g., methoxy, ethoxy, and n-propoxy groups), the aryl groups mentioned above, alkoxycarbonyl groups (e.g., methoxycarbonyl, ethoxycarbonyl, n-propoxycarbonyl, and n-butyloxycarbonyl groups), and the like.

Taking into consideration the fact that the nucleic acid delivery material of the present invention is used to deliver nucleic acids, i.e., used inside the body, it is preferable to employ substituents that are likely to improve solubility in physiological buffers, i.e., that are likely to improve water solubility. From such a viewpoint, the substituents in the nitrogen-containing heterocyclic ring represented by Ar³ in formula (2B) are preferably hydroxyl groups, alkoxy groups (e.g., methoxy, ethoxy, and n-propoxy groups), alkoxycarbonyl groups (e.g., methoxycarbonyl, ethoxycarbonyl, n-propoxycarbonyl, and n-butyloxycarbonyl groups), and the like; more preferably hydroxyl groups, alkoxycarbonyl groups (e.g., methoxycarbonyl, ethoxycarbonyl, n-propoxycarbonyl, and n-butyloxycarbonyl groups), and the like; and even more preferably alkoxycarbonyl groups (e.g., methoxycarbonyl, ethoxycarbonyl, n-propoxycarbonyl, and n-butyloxycarbonyl groups), and the like.

The number of such substituents, when present, is not particularly limited, but is preferably 1 to 10, more preferably 2 to 8, and even more preferably 3 to 6, in terms of ease of precision synthesis and ease of improving water solubility.

In formula (2A), the divalent group represented by Y is not particularly limited, and examples include divalent groups having 1 to 5, preferably 1 to 4, and more preferably 1 to 3 atoms in the main chain. Examples of the atoms constituting the main chain include nitrogen, sulfur, oxygen, and carbon atoms. In terms of ease of precision synthesis and ease of improving nucleic acid delivery efficiency, preferred are nitrogen, oxygen, and carbon atoms. The divalent group may be substituted, and examples of substituents include halogen atoms (e.g., fluorine, chlorine, iodine, and bromine atoms), oxo groups, and alkyl groups (C₁₋₄ alkyl groups, such as methyl, ethyl, and n-propyl groups), and the like. Specific examples include -O-, -OCH₃-, - OCH₂CH₃-, -OCH₂CH₂CH₃-, -CH₃O-, -CH₂CH₃O-, -CH₂CH₂CH₃O-, -S-, -CH₂-SO₂-CH₂-, -NH-SO₂-, -SO₂-NH-, -CO-, -CO-NH-CH₂-, -NH-CO-CH₂-, and the like.

In formula (2B), the hydrocarbon group represented by R⁴ is not particularly limited, and examples include alkyl groups, aryl groups, groups formed by any combination thereof (e.g., aralkyl, alkylaryl, and alkylaralkyl groups), and the like. Preferred among these are alkyl groups, in terms of ease of precision synthesis, ease of improving water solubility, and ease of improving nucleic acid delivery efficiency.

The alkyl group as the hydrocarbon group represented by R⁴ may be any of linear, branched, or cyclic (preferably linear or branched, more preferably linear). The number of carbon atoms in the alkyl group (when linear or branched) is not particularly limited, and is preferably, for example, 1 to 5, and more preferably 1 to 3. The number of carbon atoms in the alkyl group (when cyclic) is not particularly limited, and is preferably, for example, 3 to 7, and more preferably 4 to 6. Specific examples of such alkyl groups include a methyl group, an ethyl group, an n-propyl group, a propyl group, an n-butyl group, an isobutyl group, a tert-butyl group, a sec-butyl group, and the like.

The aryl group as the hydrocarbon group represented by R⁴ is not particularly limited, but is preferably one having 6 to 12 carbon atoms, and more preferably one having 6 to 8 carbon atoms. The aryl group can be monocyclic or polycyclic (e.g., bicyclic or tricyclic), but is preferably monocyclic. Specific examples of such aryl groups include a phenyl group, a naphthyl group, a biphenyl group, a pentalenyl group, an indenyl group, an anthranyl group, a tetracenyl group, a pentacenyl group, a pyrenyl group, a perylenyl group, a fluorenyl group, a phenanthryl group, and the like.

The aralkyl group as the hydrocarbon group represented by R⁴ is not particularly limited, and examples include aralkyl groups in which a hydrogen atom (e.g., 1 to 3, preferably 1 hydrogen atom) of a linear or branched alkyl group having 1 to 5 (preferably 1 to 3) carbon atoms is replaced by the aryl group mentioned above. Specific examples of such aralkyl groups include a benzyl group, a phenethyl group, and the like.

The alkylaryl group as the hydrocarbon group represented by R⁴ is not particularly limited, and examples include alkylaryl groups in which a hydrogen atom (e.g., 1 to 3, preferably 1 hydrogen atom) of the aryl group mentioned above is replaced by a linear or branched alkyl group having 1 to 5 (preferably 1 to 3) carbon atoms. Specific examples of such alkylaryl groups include a tolyl group, a xylyl group, and the like.

The alkylaralkyl group as the hydrocarbon group represented by R⁴ is not particularly limited, and examples include alkylaralkyl groups in which a hydrogen atom (e.g., 1 to 3, preferably 1 hydrogen atom) on the aromatic ring of the aralkyl group mentioned above is replaced by a linear or branched alkyl group having 1 to 5 (preferably 1 to 3) carbon atoms.

When the hydrocarbon group represented by R⁴ is substituted, examples of substituents that the hydrocarbon group may have include hydroxyl groups, alkoxy groups (e.g., methoxy, ethoxy, and n-propoxy groups), alkoxycarbonyl groups (e.g., methoxycarbonyl, ethoxycarbonyl, n-propoxycarbonyl, and n-butyloxycarbonyl groups), and the like.

The number of such substituents, when present, is not particularly limited, and can be, for example, 1 to 6, preferably 1 to 4, and more preferably 1 or 2.

Specific examples of the group having a primary, secondary, or tertiary amino group or nitrogen cation-containing group that satisfies the above conditions include:

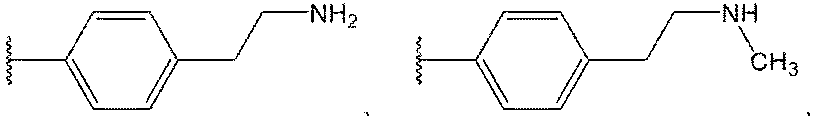

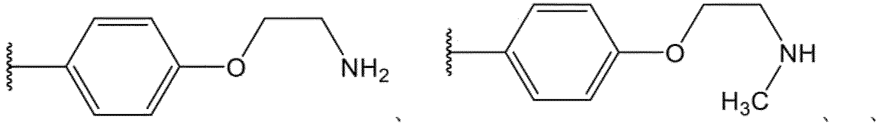

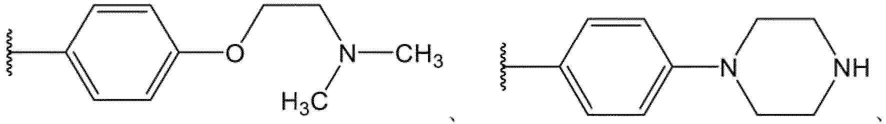

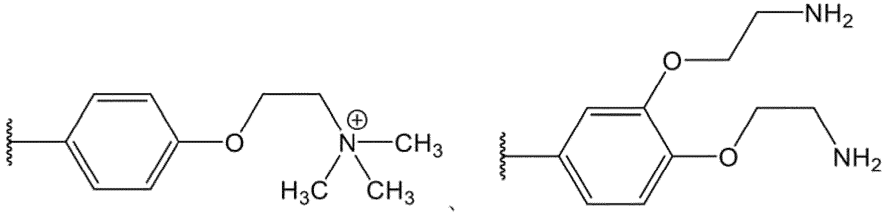

Examples of the polycyclic aromatic compound constituting the nucleic acid delivery material of the present invention that satisfies the above conditions include:

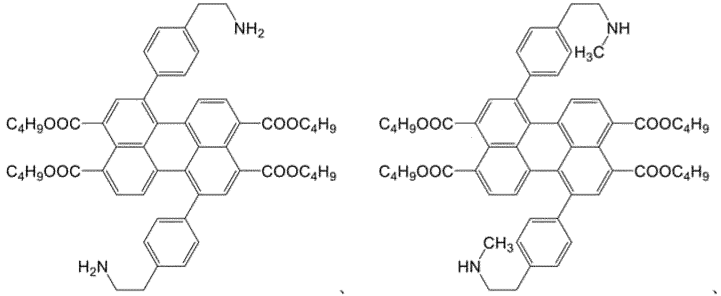

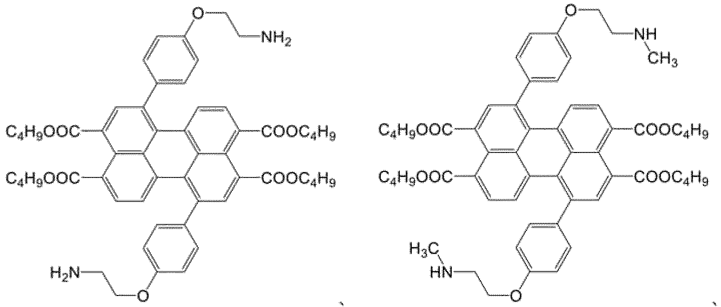

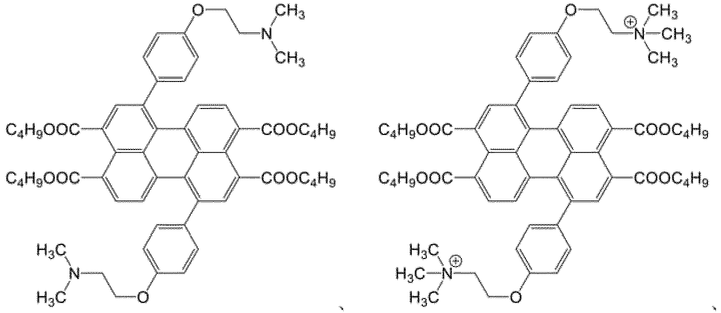

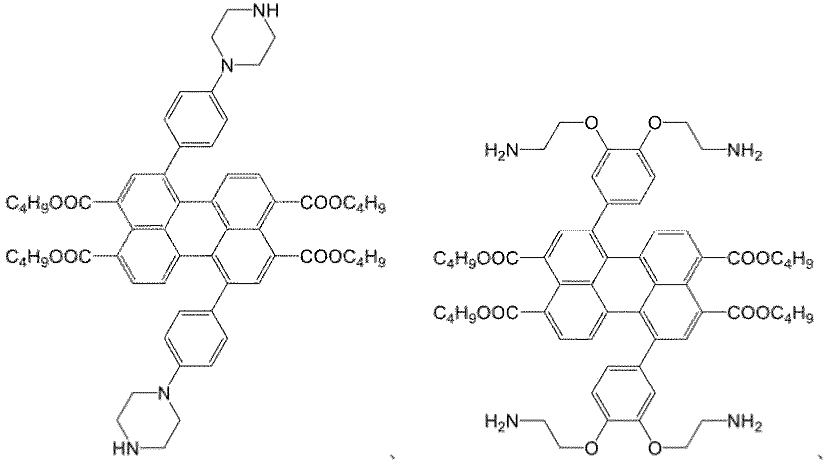

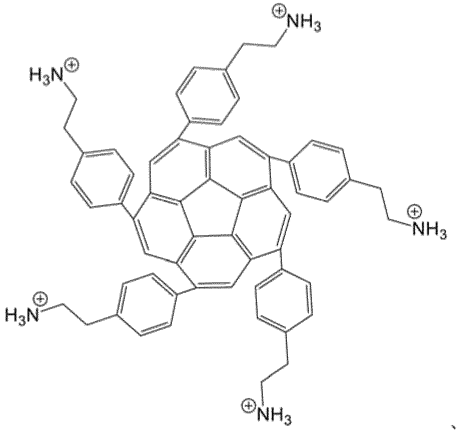

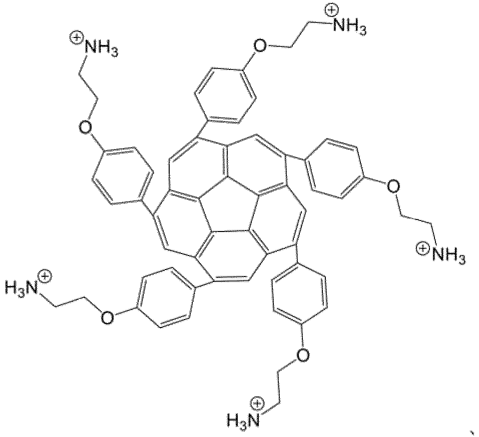

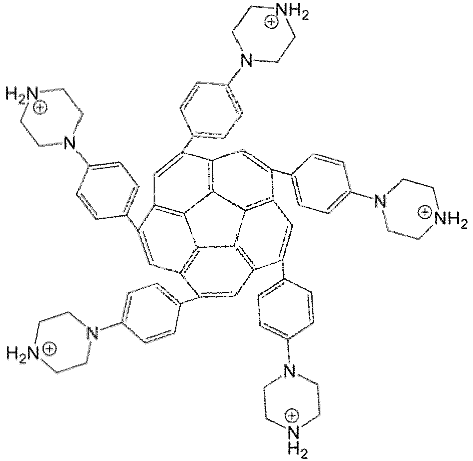

The method for producing such a polycyclic aromatic compound is not particularly limited, and the compound can be produced by various methods. For example, the polycyclic aromatic compound can be produced by reacting a compound represented by formula (3): 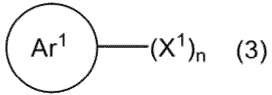 wherein Ar¹ and n are as defined above, X¹ represents a halogen atom, or boronic acid or an ester group thereof, and when n is an integer of 2 or more, n-number of X¹s may be the same or different;
with a compound represented by formula (4):

R'-Y (4)

wherein R' represents a group having an optionally protected primary, secondary or tertiary amino group or a nitrogen cation-containing group, and Y represents a halogen atom, or boronic acid or an ester group thereof.

However, when X¹ in formula (3) is a halogen atom, Y in formula (4) is preferably boronic acid or an ester group thereof, and when X¹ in formula (3) is boronic acid or an ester group thereof, Y in formula (4) is preferably a halogen atom.

In formulas (3) and (4), examples of the halogen atom represented by X¹ and Y include a chlorine atom, a bromine atom, an iodine atom, and the like.

In formulas (3) and (4), the boronic acid or an ester group thereof represented by Y is preferably a group represented by: 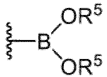 wherein two R⁵s are the same or different and each represents a hydrogen atom or an alkyl group, and two R⁵s may be bonded to each other to form a ring together with the adjacent -O-B-O-.

R⁵s in the boronic acid or an ester group thereof are hydrogen atoms or alkyl groups.

The alkyl groups may be any of those mentioned above, and the type and number of substituents are also the same.

When R⁵s are alkyl groups, the carbon atoms constituting each alkyl group may be bonded to each other to form a ring together with the adjacent -O-B-O-. In this case, the two oxygen atoms are bonded via an alkylene group (e.g., a C₁₋₁₀ alkylene group, such as a methylene group, an ethylene group, a trimethylene group, or a tetramethylene group).

Such boronic acid or an ester group thereof is, for example, a group represented by: 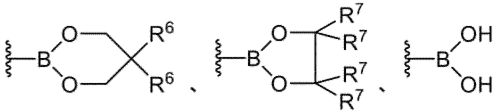 wherein R⁶ and R⁷ are the same or different and each represents a hydrogen atom or an alkyl group.

R⁶ and R⁷ are hydrogen atoms or alkyl groups.

The alkyl groups may be any of those mentioned above, and the type and number of substituents are also the same.

The boronic acid or an ester group thereof may be substituted. When the boronic acid or an ester group thereof is substituted, examples of substituents include halogen atoms (e.g., fluorine, chlorine, bromine, and iodine atoms), alkoxy groups (e.g., methoxy, ethoxy, and n-propoxy groups), the aryl groups mentioned above, the boronic acid or an ester group thereof mentioned above, a cyano group, and the like. When these substituents are present, the number thereof can be, for example, 1 to 5.

Examples of such a compound represented by formula (3) include the following:

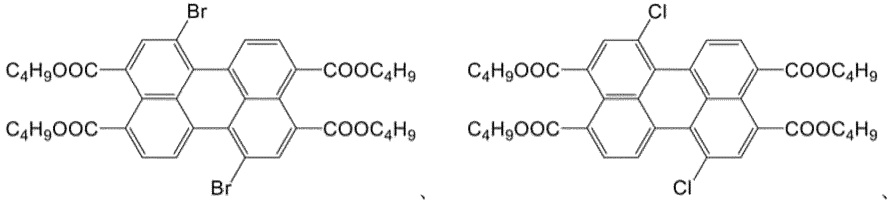

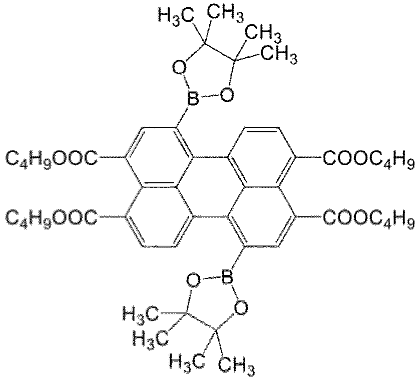

In formula (4), R' is a group having a primary, secondary or tertiary amino group or nitrogen cation-containing group, and this amino group may be protected.

When the amino group is protected, the protecting group is not particularly limited, and examples include an alkoxycarbonyl group, an acyl group, an alkylsulfonyl group, an arylsulfonyl group, and the like. These protecting groups may be substituted with the alkyl group, aryl group, aralkyl group, alkylaryl group, aralkyl group, or the like mentioned above. Among these, the protecting group for the amino group is preferably an alkoxycarbonyl group in terms of ease of synthesis, yield, and the like. Specific examples include a methoxycarbonyl group, an ethoxycarbonyl group, an n-propoxycarbonyl group, an isopropoxycarbonyl group, a tert-butyloxycarbonyl group, and the like. When the protecting group for the amino group is used, it is preferable to remove the protecting group by a conventional method after the reaction.

Examples of such a compound represented by formula (4) include the following: 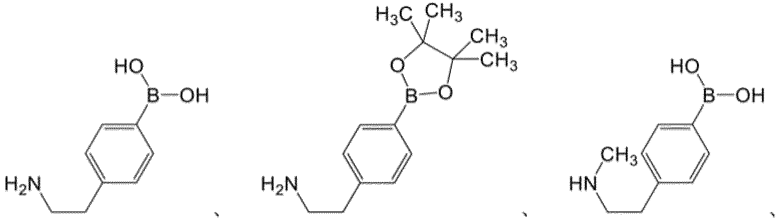 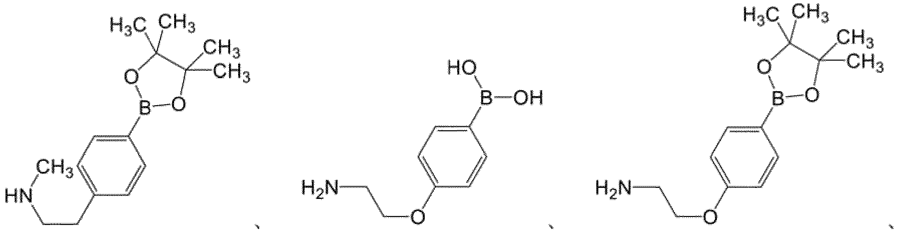 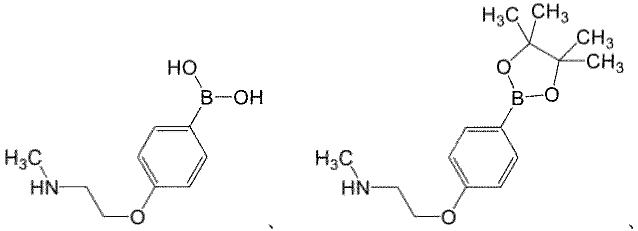 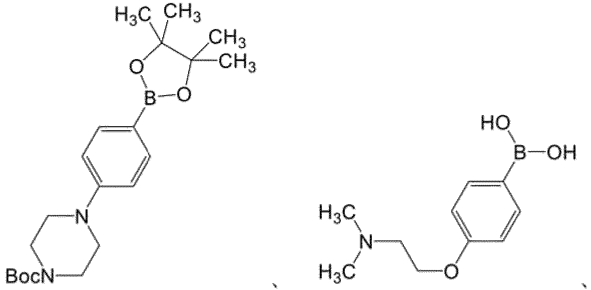 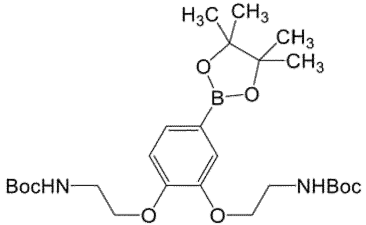 wherein Boc represents a tert-butyloxycarbonyl group.

The reaction is not particularly limited, but can be performed by Suzuki-Miyaura coupling. The reaction conditions may be those usually employed in Suzuki-Miyaura coupling.

### (1-2) Nucleic Acid Delivery Material

In the present invention, the polycyclic aromatic compound described above can be directly used as the nucleic acid delivery material of the present invention; that is, it can be used in a minimum essential medium (MEM). The nucleic acid delivery material of the present invention may further contain a solvent.

When the nucleic acid delivery material of the present invention contains a solvent, taking into consideration the fact that the nucleic acid delivery material of the present invention is used inside the body, the solvent is preferably a physiological buffer in terms of nucleic acid delivery efficiency and safety.

When a physiological buffer is used as the solvent, the pH thereof is preferably 6.0 to 8.0, more preferably 6.5 to 7.8, and even more preferably 7.0 to 7.6, in terms of nucleic acid delivery efficiency and safety.

Specific examples of such physiological buffers include Tris-buffered saline (TBS), HEPES-buffered saline (HBS), MES-buffered saline (MBS), phosphate buffer, and the like. In terms of nucleic acid delivery efficiency, preferred among these are Tris-buffered saline (TBS), HEPES-buffered saline (HBS), MES-buffered saline (MBS), etc.; more preferred are Tris-buffered saline (TBS), HEPES-buffered saline (HBS), etc.; and particularly preferred is HEPES-buffered saline (HBS).

When the nucleic acid delivery material of the present invention contains a solvent, the concentration of the polycyclic aromatic compound in the nucleic acid delivery material of the present invention is not particularly limited. In terms of nucleic acid delivery efficiency and safety, the concentration of the polycyclic aromatic compound is preferably 1 µM to 100 µM, more preferably 2.5 µM to 90 µM, even more preferably 10 µM to 80 µM, and particularly preferably 20 to 70 µM.

In addition, the nucleic acid delivery material of the present invention may also contain additives that can be conventionally contained in nucleic acid delivery materials, such as antibiotics, antifungal agents, and serum, to the extent that the effects of the present invention are not impaired.

### 2. Application

The nucleic acid delivery material of the present invention is capable of efficiently delivering nucleic acids while using small molecules that can be precisely synthesized. That is, the nucleic acid delivery material of the present invention can be used to mix with a nucleic acid, administer the nucleic acid to a living body, deliver the nucleic acid to a target tissue, and then transport the nucleic acid to cells of the target tissue. Therefore, the present invention may also include a pharmaceutical composition (nucleic acid pharmaceutical composition) prepared by incorporating a nucleic acid into the nucleic acid delivery material of the present invention.

The nucleic acid that can be delivered is not particularly limited, and a wide variety of nucleic acids can be used, including DNA, RNA, natural or non-natural nucleic acid analogs (e.g., peptide nucleic acids), altered nucleic acids, modified nucleic acids, and the like. In addition, the nucleic acid may be single-stranded or double-stranded, and the presence or absence of protein encoding or other functions is also not limited.

Among them, the nucleic acid is preferably a functional nucleic acid that can exert some action on the living body, tissues, cells, etc. when delivered into the living body. Examples of functional nucleic acids include plasmid DNA, siRNA, micro-RNA (miRNA), antisense RNA, antisense DNA, decoy nucleic acid, ribozyme, DNA enzyme, various inhibitory genes (e.g., tumor suppressor genes), functional altered nucleic acid/modified nucleic acid (e.g., nucleic acid whose phosphate portion has been altered to phosphorothioate, methylphosphonate, phosphate triester, phosphoramidate, etc., and nucleic acid to which a hydrophobic functional group, such as cholesterol or vitamin E, is bound), and the like. These are selected depending on the application of the nucleic acid delivery material of the present invention.

The plasmid DNA is not limited as long as it can exert a desired function in a target cell or tissue. Various types of plasmid DNA are known, and a person skilled in the art could have selected desired plasmid DNA depending on the application of the nucleic acid delivery material of the present invention. For example, the target cell or tissue is not particularly limited; however, delivery to various mammalian cells, plant cells, and intestinal bacteria (e.g., *E. coli*) is possible.

siRNA is not limited as long as it is capable of inhibiting the expression of a target gene by taking advantage of RNA interference (RNAi). Preferred examples of target genes for RNA interference include cancer (tumor) genes, anti-apoptotic genes, cell cycle-related genes, growth signal genes, and the like. The base length of siRNA is not limited, but is generally less than 30 bases, and preferably 10 to 25 bases.

In the present invention, the nucleic acid is preferably DNA, and more preferably plasmid DNA, in terms of nucleic acid delivery efficiency.

The nucleic acid delivery material of the present invention can be used as a pharmaceutical composition (nucleic acid pharmaceutical composition) by mixing with a nucleic acid to form a complex. When a pharmaceutical composition is prepared, a nucleic acid can form a complex with a polycyclic aromatic compound through electrostatic interactions. Therefore, the amount of nucleic acid is not particularly limited, but can be set according to a conventional method.

The pharmaceutical composition (nucleic acid pharmaceutical composition) of the present invention can be used to deliver a nucleic acid to a target cell or tissue in vitro or in vivo. According to the pharmaceutical composition (nucleic acid pharmaceutical composition) of the present invention, a nucleic acid can be easily delivered in a stabilized state into a target cell.

To deliver a nucleic acid to a target cell or tissue using the pharmaceutical composition (nucleic acid pharmaceutical composition) of the present invention, the pharmaceutical composition (nucleic acid pharmaceutical composition) can be brought into a state in which it can come into contact with the target cell or tissue.

To achieve in vitro contact between the pharmaceutical composition (nucleic acid pharmaceutical composition) of the present invention and a target cell or tissue, the target cell or tissue can be cultured in the presence of the pharmaceutical composition (nucleic acid pharmaceutical composition) of the present invention, or the pharmaceutical composition (nucleic acid pharmaceutical composition) of the present invention can be added to a culture of the target cell or tissue.

To achieve in vivo contact between the pharmaceutical composition (nucleic acid pharmaceutical composition) of the present invention and a target cell or tissue, the pharmaceutical composition (nucleic acid pharmaceutical composition) of the present invention can be administered to an individual in need of introduction of the nucleic acid (or an individual to be treated) by an administration method commonly used in the art, such as gene therapy. Examples of such individuals include, but are not limited to, humans, mice, rats, rabbits, dogs, cats, monkeys, cows, horses, pigs, and other mammals. Methods of administration include direct introduction or implantation near or within the target cell or tissue, intravenous injection, arterial injection, intramuscular injection, oral administration, pulmonary administration, and the like. The dosage, administration frequency, administration period, and other conditions can be appropriately set according to the type, condition, etc. of the subject animal.

Diseases to be treated with the pharmaceutical composition (nucleic acid pharmaceutical composition) of the present invention are not particularly limited as long as they are caused by inappropriate functional expression of genes. Examples include cancers (lung cancer, pancreatic cancer, brain tumors, liver cancer, breast cancer, colorectal cancer, neuroblastoma, bladder cancer, etc.), cardiovascular diseases, musculoskeletal diseases, central nervous system diseases, and the like.

The pharmaceutical composition of the present invention may contain other additives that are commonly used in pharmaceutical preparations. Examples of other additives include excipients, extenders, fillers, binders, wetting agents, disintegrants, lubricants, surfactants, dispersants, buffers, preservatives, solubilizing agents, antiseptics, flavoring agents, soothing agents, stabilizers, isotonic agents, and the like. Such additives may be used singly or in any combination of two or more in any ratio. The details of these other components, such as the type and amount used, can be appropriately determined by a person skilled in the art depending on the purpose, application, method of use, etc. of the pharmaceutical composition.

The pharmaceutical composition of the present invention may take any form, but is usually provided as an intravenous injection (including drip infusion) in the form of, for example, a unit dose ampoule or other dosage container.

The pharmaceutical composition of the present invention may be used in any manner. A pharmaceutical composition containing the nucleic acid delivery material of the present invention can be administered as is.

### 3. Compound

As described above, among the polycyclic aromatic compounds (in particular, the compounds represented by formula (1)) used for the nucleic acid delivery material of the present invention, the compound represented by formula (1'): 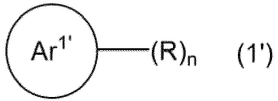
wherein Ar^{1'} represents an optionally substituted fused aromatic ring having 4 or more rings other than a pyrene ring and a perylene diimide ring, R represents a group having a primary, secondary, or tertiary amino group or nitrogen cation-containing group, n represents an integer of 1 or more, and when n is an integer of 2 or more, n Rs may be the same or different, provided that when Ar¹' is a perylene ring, n Rs bind to n-number of positions 1, 2, 5, 6, 7, 8, 11, and 12 in Ar¹';
is a novel compound that has not been described in any literature.

In formula (1'), R and n can be as defined above.

Therefore, the polycyclic aromatic compound used for the nucleic acid delivery material of the present invention is preferably a compound represented by formula (1A'): 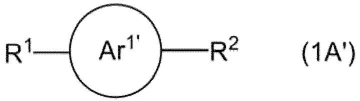 wherein Ar¹' represents an optionally substituted fused aromatic ring having 4 or more rings other than a pyrene ring and a perylene diimide ring, and R¹ and R² are the same or different and each represents a group having a primary, secondary, or tertiary amino group or nitrogen cation-containing group, provided that when Ar¹' is a perylene ring, R¹ and R² bind to two of positions 1, 2, 5, 6, 7, 8, 11, and 12 in Ar¹'.

In formulas (1') and (1A'), the fused aromatic ring represented by Ar¹' refers to a fused aromatic ring having 4 or more rings other than a pyrene ring and a perylene diimide ring, among the fused aromatic rings represented by Ar¹' shown above. The number of rings contained in the fused aromatic ring represented by Ar¹' is not particularly limited, but is preferably 5 to 10, more preferably 5 to 8, and even more preferably 5 to 6, in terms of ease of precision synthesis and ease of improving nucleic acid delivery efficiency.

Specific examples of the fused aromatic ring represented by Ar¹' include a tetracene ring, a chrysene ring, a triphenylene ring, a pentacene ring, a perylene ring, a benzopyrene ring, a coronene ring, a corannulene ring, and the like.

The fused aromatic ring represented by Ar¹' in formulas (1') and (1A') may be substituted. Examples of substituents include, but are not particularly limited to, hydroxyl groups, halogen atoms (e.g., fluorine, chlorine, bromine, and iodine atoms), alkyl groups (e.g., methyl, ethyl, and n-propyl groups), alkoxy groups (e.g., methoxy, ethoxy, and n-propoxy groups), the aryl groups mentioned above, alkoxycarbonyl groups (e.g., methoxycarbonyl, ethoxycarbonyl, n-propoxycarbonyl, and n-butyloxycarbonyl groups), and the like.

Taking into consideration the fact that the compound of the present invention is used to deliver nucleic acids, i.e., used inside the body, it is preferable to employ substituents that are likely to improve solubility in physiological buffers, i.e., that are likely to improve water solubility. From such a viewpoint, the substituents in the fused aromatic ring presented by Ar¹ in formulas (1') and (1A') are preferably hydroxyl groups, alkoxy groups (e.g., methoxy, ethoxy, and n-propoxy groups), alkoxycarbonyl groups (e.g., methoxycarbonyl, ethoxycarbonyl, n-propoxycarbonyl, and n-butyloxycarbonyl groups), and the like; more preferably hydroxyl groups, alkoxycarbonyl groups (e.g., methoxycarbonyl, ethoxycarbonyl, n-propoxycarbonyl, and n-butyloxycarbonyl groups), and the like; and even more preferably alkoxycarbonyl groups (e.g., methoxycarbonyl, ethoxycarbonyl, n-propoxycarbonyl, and n-butyloxycarbonyl groups), and the like.

The number of such substituents, when present, is not particularly limited, but is preferably 1 to 10, more preferably 2 to 8, and even more preferably 3 to 6, in terms of ease of precision synthesis and ease of improving water solubility.

Examples of the compound having such a fused aromatic ring of the present invention include the compound represented by formula (1A1) or (1A2) described above.

### Examples

The present invention is described in detail below with reference to Examples etc.; however, the present invention is not limited thereto.

### Synthesis Example 1: Synthesis of KTU059

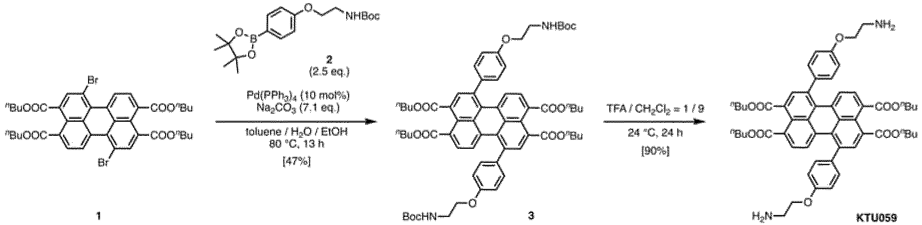

Compound 1 (206 mg, 254 µmol) (Reference 1), compound 2 (228 mg, 628 µmol) (Reference 2), Pd(PPh₃)₄ (29.9 mg, 25.9 µmol), and Na₂CO₃ (192 mg, 1.81 mmol) were dissolved in toluene (3.0 mL), water (1.0 mL), and ethanol (0.5 mL). The mixture was stirred under a nitrogen atmosphere at 80°C for 13 hours. Thereafter, the resulting solids were removed by filtration, and extraction operations were performed three times with dichloromethane (20 mL). The resulting organic layer was washed with saturated saline, dried over Na₂SO₄, and distilled under reduced pressure to obtain a crude product. The resulting crude product was purified by preparative thin-layer chromatography to obtain compound 3 (135 mg, 47%).
¹H NMR (600 MHz, CD₂Cl₂) δ 8.01 (s, 2H), 7.61 (d, J = 7.8 Hz, 2H), 7.56 (d, J = 7.8 Hz, 2H), 7.44 (d, J = 8.4 Hz, 4H), 6.98 (d, J = 9.0 Hz, 4H), 5.06 (br, 2H), 4.28 (t, 6.6 Hz, 4H), 4.24 (t, 6.6 Hz, 4H), 4.08 (t, J = 4.8 Hz, 4H), 3.55 (d, J = 4.8 Hz, 4H), 1.77-1.70 (m, 8H), 1.49-1.41 (m, 26H), 0.98-0.95 (m, 12H); MS (MALDI-TOF MS) m/z calc. for C₆₆H₇₈N₂O₁₄ [M]⁺: 1122.5448, found: 1122.5446.

Compound 3 (3.4 mg, 5.7 µmol) was dissolved in 0.9 mL of dichloromethane. 0.1 mL of TFA was added, followed by stirring at 24°C for 24 hours. Thereafter, 6 mL of 1 M aqueous sodium hydroxide solution was added, and extraction operations were performed three times with dichloromethane (10 mL). The resulting organic layer was washed with saturated saline, dried over Na₂SO₄, and distilled under reduced pressure to obtain KTU059 (15.1 mg, 90%) .
¹H NMR (600 MHz, CDCl₃) δ 8.02 (s, 2H), 7.58 (d, J = 7.8 Hz, 2H), 7.57 (d, J = 8.4 Hz, 2H), 7.40 (d, J = 9.0 Hz, 4H), 6.96 (d, J = 8. 4 Hz, 4H), 4.32 (t, 7.2 Hz, 4H), 4.28 (t, 7.2 Hz, 4H), 4.07 (t, J = 4.8 Hz, 4H), 3.15 (t, J = 4.8 Hz, 4H), 1.75 (m, 8H), 1.49-1.42 (m, 8H), 0.99-0.96 (m, 12H); MS (MALDI-TOF MS) m/z calc. for C₅₆H₆₂N₂O₁₀ [M]⁺: 922.4399, found: 922.4399.

### Synthesis Example 2: Synthesis of KTU141

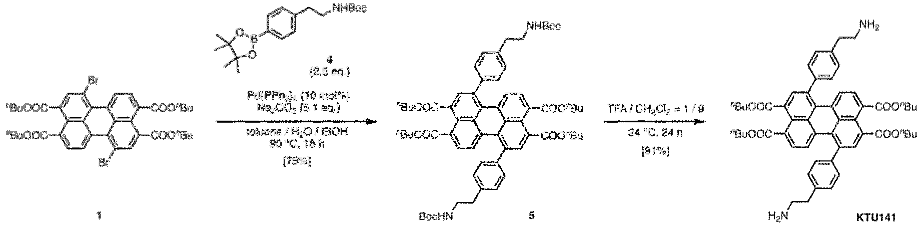

Compound 1 (150 mg, 185 µmol) (Reference 1), compound 4 (158 mg, 456 µmol) (Reference 3), Pd(PPh₃)₄ (24.4 mg, 21.1 µmol), and Na₂CO₃ (101 mg, 0.953 mmol) were dissolved in toluene (3.0 mL), water (1.0 mL), and ethanol (0.5 mL). The mixture was stirred under a nitrogen atmosphere at 90°C for 18 hours. Thereafter, the resulting solids were removed by filtration, and extraction operations were performed three times with dichloromethane (20 mL). The resulting organic layer was washed with saturated saline, dried over Na₂SO₄, and distilled under reduced pressure to obtain a crude product. The resulting crude product was purified by preparative thin-layer chromatography to obtain compound 5 (163 mg, 80%).
¹H NMR (500 MHz, CD₂Cl₂) δ 8.03 (s, 2H), 7.58 (d, J = 8.0 Hz, 2H), 7.55 (d, J = 8.0 Hz, 2H), 7.46 (d, J = 8.0 Hz, 2H), 7.30 (d, J = 8.0 Hz, 2H), 4.69 (br, 2H), 4.29 (t, J = 6.9 Hz, 4H), 4.25 (t, J = 6.9 Hz, 4H), 3.41 (d, J = 6.5 Hz, 4H), 2.86 (t, J = 7.0 Hz, 4H), 1.78-1.69 (m, 8H), 1.50-1.40 (m, 26H), 0.99-0.95 (m, 12H).

Compound 5 (21.0 mg, 19.2 µmol) was dissolved in 0.9 mL of dichloromethane. 0.1 mL of TFA was added, followed by stirring at 24°C for 24 hours. Thereafter, 6 mL of 1 M aqueous sodium hydroxide solution was added, and extraction operations were performed three times with dichloromethane (10 mL). The resulting organic layer was washed with saturated saline, dried over Na₂SO₄, and distilled under reduced pressure to obtain KTU141 (15.6 mg, 91%) .
¹H NMR (600 MHz, CD₂Cl₂) δ 8.03 (s, 2H), 7.58 (d, J = 7.8 Hz, 1H), 7.52 (d, J = 7.8 Hz, 1H), 7.45 (d, J = 7.8 Hz, 4H), 7.30 (d, J = 8.4 Hz, 4H), 4.28 (t, J = 6.8 Hz, 4H), 4.24 (t, J = 6.8 Hz, 4H), 3.00 (d, J = 6.6 Hz, 4H), 2.81 (t, J = 7.2 Hz, 4H), 1.77-1.69 (m, 8H), 1.49-1.41 (m, 8H), 0.99-0.95 (m, 12H).

### Synthesis Example 3: Synthesis of KTU048

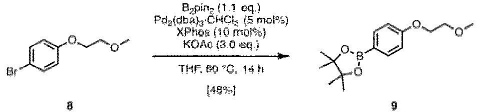

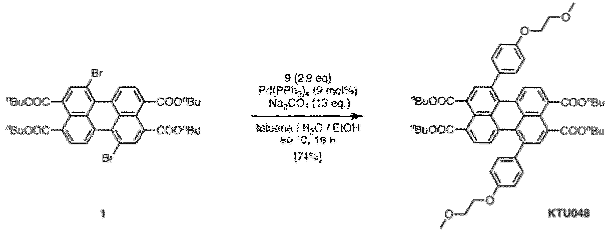

Compound 8 (342 mg, 1.48 mmol) (Reference 4), bispinacolatodiboron (B₂pin₂) (423 mg, 1.67 mmol), Pd₂dba₃·CHCl₃ (70.6 mg, 77.1 µmol), XPhos (72.6 mg, 152 µmol), and KOAc (435 mg, 4.44 mmol) were dissolved in THF (10 mL). The mixture was stirred under a nitrogen atmosphere at 60°C for 14 hours. Thereafter, the resulting solids were removed by filtration, water was added, and extraction operations were performed three times with ethyl acetate (20 mL). The resulting organic layer was washed with saturated saline, dried over Na₂SO₄, and distilled under reduced pressure to obtain a crude product. The resulting crude product was purified by column chromatography to obtain compound 9 (197 mg, 48%).
¹H-NMR (600 MHz, CDCl₃) δ 7.74 (d, J = 8.6 Hz, 2H), 6.91 (d, J = 8.6 Hz, 2H), 4.13-4.12 (m, 2H), 3.74-3.73 (m, 2H), 3.43 (s, 3H), 1.32 (s, 12H).

Compound 1 (50.3 mg, 61.8 µmol) (Reference 1), compound 9 (50.0 mg, 180 µmol), Pd(PPh₃)₄ (6.70 mg, 5.80 µmol), and Na₂CO₃ (83.2 mg, 0.785 mmol) were dissolved in toluene (3.0 mL), water (1.1 mL), and ethanol (0.5 mL). The mixture was stirred under a nitrogen atmosphere at 80°C for 16 hours. Thereafter, the resulting solids were removed by filtration, and extraction operations were performed three times with dichloromethane (20 mL). The resulting organic layer was washed with saturated saline, dried over Na₂SO₄, and distilled under reduced pressure to obtain a crude product. The resulting crude product was purified by preparative thin-layer chromatography and gel filtration chromatography to obtain KTU048 (43.5 mg, 74%).
¹H NMR (600 MHz, CDCl₃) δ 8.02 (s, 2H), 7.56 (s, 4H), 7.39 (d, J = 8.9 Hz, 4H), 6.98 (d, J = 8.9 Hz, 4H), 4.32 (t, J = 6.6 Hz, 4H), 4.29 (t, J = 6.6 Hz, 4H), 4.21-4.17 (m, 4H), 3.82-3.79 (m, 4H), 3.49 (s, 6H), 1.79-1.73 (m, 8H), 1.49-1.43 (m, 8H), 0.99-0.96 (m, 12H) .

### Synthesis Example 4: Synthesis of KTU099

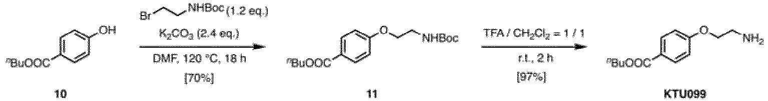

Compound 10 (199 mg, 1.02 mmol) and K₂CO₃ (364 mg, 2.63 mmol) were dissolved in DMF (10 mL). Thereafter, 2-(tert-butoxycarbonylamino)ethyl bromide (271 mg, 1.21 mmol) was added dropwise at room temperature. After the addition was completed, the mixture was stirred at 120°C for 18 hours. Thereafter, water was added, and extraction operations were performed three times with ethyl acetate (20 mL). The resulting organic layer was washed with saturated saline, dried over Na₂SO₄, and distilled under reduced pressure to obtain a crude product. The resulting crude product was purified by column chromatography to obtain compound 11 (237 mg, 70%).
¹H NMR (500 MHz, CDCl₃) δ 7.98 (d, J = 8.5 Hz, 2H), 6.90 (d, J = 9.0 Hz, 2H), 5.02 (br, 1H), 4.28 (t, J = 7.0 Hz, 2H), 4.06 (t, J = 5.0 Hz, 2H), 3.60-3.49 (m, 2H), 1.77-1.71 (m, 2H), 1.51-1.43

### (m, 11H), 0.97 (t, J = 6.0 Hz, 3H).

Compound 11 (51.6 mg, 153 µmol) was dissolved in 0.4 mL of dichloromethane. 0.4 mL of TFA was added, followed by stirring at room temperature for 2 hours. Thereafter, 6 mL of 1 M aqueous sodium hydroxide solution was added, and extraction operations were performed three times with dichloromethane (10 mL). The resulting organic layer was washed with saturated saline, dried over Na₂SO₄, and distilled under reduced pressure to obtain KTU099 (35.1 mg, 97%).
¹H NMR (500 MHz, CDCl₃) δ 7.98 (d, J = 9.0 Hz, 2H), 6.92 (d, J = 9.0 Hz, 2H), 4.28 (t, J = 7.0 Hz, 2H), 4.03 (t, J = 5.5 Hz, 2H), 3.20-3.03 (m, 2H), 1.77-1.71 (m, 2H), 1.50-1.44 (m, 2H), 0.98 (t, J = 7.5 Hz, 3H).

### Synthesis Example 5: Synthesis of DZ018

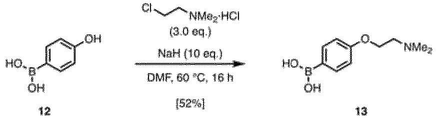

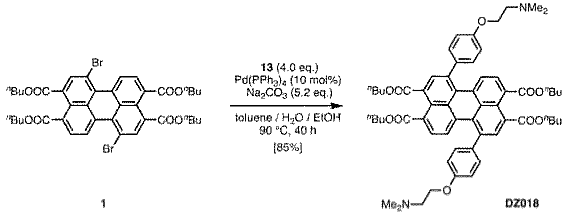

Compound 12 (50.0 mg, 362 µmol), 2-(dimethylamino)ethyl chloride hydrochloride (156 mg, 1.09 mmol), and NaH (60% oil dispersion) (145 mg, 3.63 mmol) were dissolved in DMF (3.6 mL), followed by stirring at 60°C for 16 hours. Thereafter, a saturated aqueous ammonium chloride solution was added, and extraction operations were performed three times with dichloromethane. The resulting organic layer was washed with saturated saline, dried over MgSO₄, and distilled under reduced pressure to obtain a crude product. The resulting crude product was purified by preparative thin-layer chromatography to obtain compound 13 (41.0 mg, 54%).

Compound 1 (200 mg, 247 µmol) (Reference 1), compound 13 (206 mg, 987 µmol), Pd(PPh₃)₄ (28.5 mg, 25.0 µmol), and Na₂CO₃ (134 mg, 1.27 mmol) were dissolved in toluene (6 mL), water (2 mL), and ethanol (1 mL). The mixture was stirred under a nitrogen atmosphere at 90°C for 40 hours. After the reaction, extraction operations were performed three times with dichloromethane (10 mL). The resulting organic layer was dried over MgSO₄ and distilled under reduced pressure to obtain a crude product. The resulting crude product was purified by column chromatography to obtain compound DZ018 (205 mg, 85%).
¹H NMR (600 MHz, CDCl₃) δ 8.02 (s, 2H), 7.560 (s, 2H), 7.556 (s, 2H), 7.39 (d, J = 9.0 Hz, 4H), 6.97 (d, J = 8.4 Hz, 4H), 4.31 (t, J = 6.6 Hz, 4H), 4.23 (t, J = 6.6 Hz, 4H), 4.13 (t, J = 6.0 Hz, 4H), 2.78 (t, J = 5.4 Hz, 4H), 2.377 (s, 12H), 1.79-1.70 (m, 8H), 1.48-1.40 (m, 8H), 0.98-0.95 (m, 12H).

### Synthesis Example 6: Synthesis of DZ023

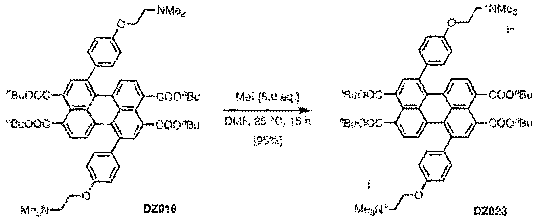

DZ018 (31.0 mg, 31.6 µmol) was dissolved in 1 mL of acetonitrile. Methyl iodide (10 µL, 158.3 µmol) was added, followed by stirring at 25°C for 15 hours. Thereafter, the solvent was distilled under reduced pressure, and the resulting crude product was washed with acetone and hexane to obtain DZ023 (38.0 mg, 95%).
¹H NMR (600 MHz, CD₃OD) δ 7.82 (s, 2H), 7.35-7.27 (m, 4H), 7.07 (J = 9.0 Hz, 4H), 7.04-6.98 (m, 4H), 4.36-4.31(m, 8H), 3.94-3.89 (m, 4H), 3.88-3.84 (m, 4H), 3.29 (s, 18H), 1.83-1.71 (m, 8H), 1.56-1.46 (m, 8H), 1.05-0.99 (m, 12H).

### Synthesis Example 7: Synthesis of KTU129

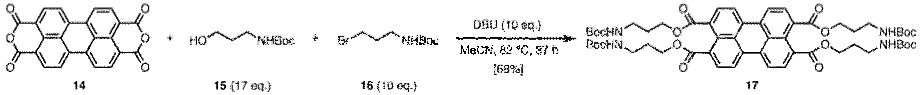

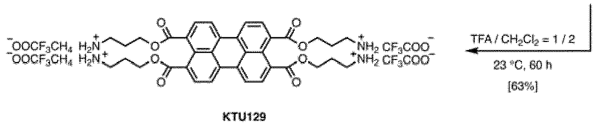

Compound 14 (208 mg, 503 µmol), compound 15 (1.58 g, 9.01 mmol), compound 16 (1.29 g, 5.38 mmol), and DBU (0.76 mL, 5.30 mmol) were dissolved in acetonitrile (5 mL), followed by stirring at 82°C for 37 hours. Thereafter, water was added, and extraction operations were performed three times with dichloromethane (20 mL). The resulting organic layer was washed with saturated saline, dried over Na₂SO₄, and distilled under reduced pressure to obtain a crude product. The resulting crude product was purified by column chromatography to obtain compound 17 (366 mg, 68%).
¹H NMR (500 MHz, CDCl₃) δ7.96 (d, J = 8.0 Hz, 4H), 7.84 (d, J = 7.5 Hz, 4H), 5.36 (br, 4H), 4.44 (t, J = 5.5 Hz, 8H), 3.42-3.28 (m, 8H), 2.06-2.03 (m, 8H), 1.45 (s, 36H).

Compound 17 (129 mg, 10.5 µmol) was dissolved in 1.2 mL of dichloromethane. 0.6 mL of TFA was added, followed by stirring at 23°C for 60 hours. Thereafter, the solvent was distilled under reduced pressure to obtain KTU129 (85.0 mg, 63%).
¹H NMR (600 MHz, D₂O) δ 7.04 (brs, 4H), 6.63 (brs, 4H), 4.26 (t, J = 6.0 Hz, 8H), 3.11 (t, J = 7.2 Hz, 8H), 2.13-2.08 (m, 8H).

### Synthesis Example 8: Synthesis of KTU131

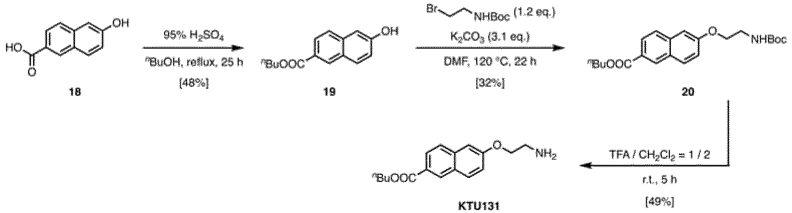

Compound 18 (1.50 g, 7.96 mmol) was dissolved in ⁿBuOH (8 mL), and 95% sulfuric acid (0.15 mL, 2.80 mmol) was added dropwise while cooling to 0°C. After the dropwise addition was completed, the mixture was stirred at 120°C for 25 hours. A saturated aqueous sodium hydrogen carbonate solution (15 mL) was added, and extraction operations were performed three times with dichloromethane (20 mL). The resulting organic layer was washed with saturated saline, dried over Na₂SO₄, and distilled under reduced pressure to obtain a crude product. The resulting crude product was purified by column chromatography to obtain compound 17 (924 mg, 48%).
1H-NMR (600 MHz, CDCl₃) δ 8.53 (s, 1H), 8.02 (dd, J = 8.4, 1.8 Hz, 1H), 7.87 (d, J = 9.0 Hz, 1H), 7.70 (d, J = 9.0 Hz, 1H), 7.19-7.14 (m, 2H), 4.37 (t, J = 6.6 Hz, 2H), 1.82-1.77 (m, 2H), 1.55-1.48 (m, 2H), 1.00 (t, J = 7.8 Hz, 3H).

Compound 19 (404 mg, 1.65 mmol), 2-(tert-butoxycarbonylamino) ethyl bromide (441 mg, 1.97 mmol), and K₂CO₃ (702 mg, 5.08 mmol) were dissolved in DMF (16 mL), followed by stirring at 120°C for 22 hours. Thereafter, water was added, and extraction operations were performed three times with a mixed solution of hexane and ethyl acetate (2:1, 20 mL). The resulting organic layer was washed with saturated saline, dried over Na₂SO₄, and distilled under reduced pressure to obtain a crude product. The resulting crude product was purified by column chromatography and preparative thin-layer chromatography to obtain compound 20 (208 mg, 32%).
¹H NMR (500 MHz, CDCl₃) δ 8.51 (s 1H), 8.02 (dd, J = 8.5, 1.5 Hz, 1H), 7.85 (d, J = 9.0 Hz, 1H), 7.73 (d, J = 8.5 Hz, 1H), 7.20-7.12 (m, 2H), 5.04 (br, 1H), 4.37 (t, J = 6.5 Hz, 2H), 4.16 (t, J = 5.0 Hz, 2H), 3.63-3.56 (m, 2H), 1.82-1.76 (m, 2H), 1.55-1.49 (m, 11H), 1.00 (t, J = 7.5 Hz, 3H).

Compound 20 (113 mg, 292 µmol) was dissolved in 2.0 mL of dichloromethane. 1 mL of TFA was added, followed by stirring at 23°C for 5 hours. Thereafter, 10 mL of 1M aqueous sodium hydroxide solution was added, and extraction operations were performed three times with dichloromethane (10 mL). The resulting organic layer was washed with saturated saline, dried over Na₂SO₄, and distilled under reduced pressure to obtain a crude product. The resulting crude product was purified by preparative thin-layer chromatography to obtain KTU131 (41.0 mg, 49%).
¹H NMR (600 MHz, CDCl₃) δ 8.52 (s, 1H), 8.03 (dd, J = 8.4, 1.2 Hz, 1H), 7.86 (d, J = 9.0 Hz, 1H), 7.74 (d, J = 8.4 Hz, 1H), 7.23 (dd, J = 9.0, 2.4 Hz, 1H), 7.16 (d, J = 2.4 Hz, 1H), 4.37 (t, J = 6.6 Hz, 2H), 4.14 (t, J = 4.8 Hz, 2H), 3.17 (t, J = 5.4 Hz, 2H), 1.82-1.77 (m, 2H), 1.55-1.48 (m, 2H), 1.01 (t, J = 7.2 Hz, 3H).

### Synthesis Example 9: Synthesis of KTU207

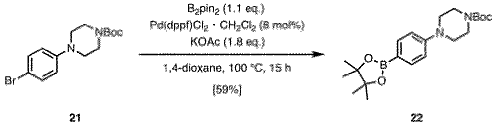

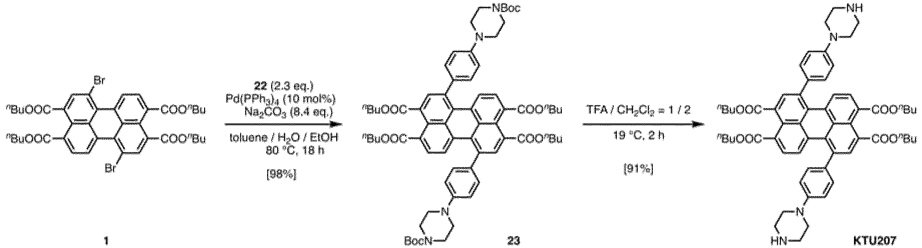

Compound 21 (502 mg, 1.14 mmol), bispinacolatodiboron (B₂pin₂) (398 mg, 1.66 mmol), Pd (dppf)Cl₂·CH₂Cl₂ (132 mg, 162 µmol), and KOAc (329 mg, 3.35 mmol) were dissolved in 1,4-dioxane (15 mL). The mixture was stirred under a nitrogen atmosphere at 100°C for 15 hours. Thereafter, the resulting solids were removed by filtration, water was added, and extraction operations were performed three times with ethyl acetate (20 mL). The resulting organic layer was washed with saturated saline, dried over Na₂SO₄, and distilled under reduced pressure to obtain a crude product. The resulting crude product was purified by column chromatography to obtain compound 22 (342 mg, 59%).
¹H NMR (600 MHz, CDCl₃) δ 7.71 (d, J = 8.4 Hz, 2H), 6.88 (d, J = 9.0 Hz, 2H), 3.60-3.55 (m, 4H), 3.25-3.18 (s, 4H), 1.48 (s, 9H), 1.32 (s, 12H).

Compound 1 (102 mg, 125 µmol) (Reference 1), compound 22 (110 mg, 284 µmol), Pd(PPh₃)₄ (15.0 mg, 13.0 µmol), and Na₂CO₃ (112 mg, 1.05 mmol) were dissolved in toluene (1.2 mL), water (0.6 mL), and ethanol (0.3 mL). The mixture was stirred under a nitrogen atmosphere at 80°C for 18 hours. Thereafter, the resulting solids were removed by filtration, and extraction operations were performed three times with dichloromethane (20 mL). The resulting organic layer was washed with saturated saline, dried over Na₂SO₄, and distilled under reduced pressure to obtain a crude product. The resulting crude product was purified by preparative thin-layer chromatography to obtain compound 23 (144 mg, 98%).
¹H NMR (600 MHz, CDCl₃) δ 8.02 (s, 2H), 7.64 (d, J = 7.8 Hz, 2H), 7.56 (d, J = 7.8 Hz, 2H), 7. 39 (d, J = 8.4 Hz, 4H), 6.95 (d, J = 8.4 Hz, 4H), 4.31 (t, J = 6.6 Hz, 4H), 4.28 (t, J = 6.6 Hz, 4H),3.65-3.60 (m, 8H), 3.24 (br, 8H), 1.78-1.71 (m, 8H), 1.50-1.43 (m, 26H), 0.99-0.95 (m, 12H); MS (MALDI-TOF MS) m/z calc. for C₇₀H₈₄N₄O₁₂ [M]⁺: 1172.61, found: 1172.62.

Compound 23 (13.0 mg, 10.5 µmol) was dissolved in 1.0 mL of dichloromethane. 0.5 mL of TFA was added, followed by stirring at 19°C for 2 hours. Thereafter, 6 mL of 1 M aqueous sodium hydroxide solution was added, and extraction operations were performed three times with dichloromethane (10 mL). The resulting organic layer was washed with saturated saline, dried over Na₂SO₄, and distilled under reduced pressure to obtain KTU207 (9.1 mg, 91%).
¹H NMR (600 MHz, CD₂Cl₂) δ 8.02 (s, 2H), 7.69 (d, J = 7.8 Hz, 2H), 7.55 (d, J = 7.8 Hz, 2H), 7.39 (d, J = 9.0 Hz, 4H), 6.96 (d, J = 8.4 Hz, 4H), 4.28 (t, J = 7.2 Hz, 4H), 4.24 (t, J = 6.6 Hz, 4H), 3.22-3.18 (m, 8H), 3.04-2.98 (m, 8H), 1.77-1.70 (m, 8H), 1.48-1.43 (m, 8H), 0.98-0.95 (m, 12H).

### Synthesis Example 10: Synthesis of KTU208

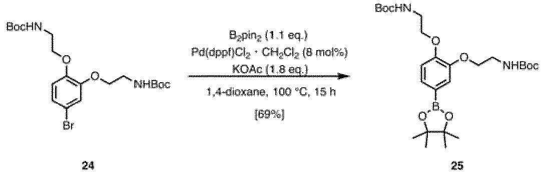

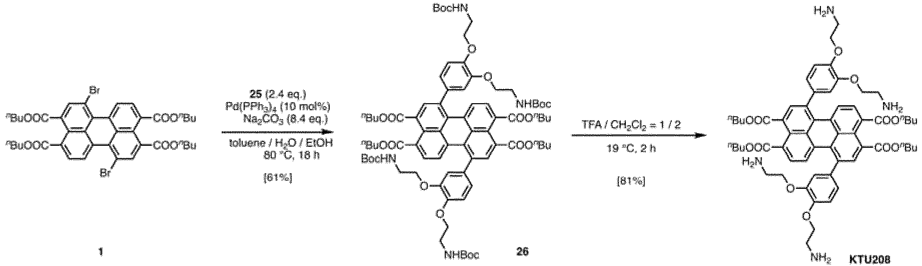

Compound 24 (412 mg, 832 µmol) (Reference 5), bispinacolatodiboron (B₂pin₂) (200 mg, 867 µmol), Pd(dppf)Cl₂·CH₂Cl₂ (60.2 mg, 73.6 µmol), and KOAc (157 mg, 1.60 mmol) were dissolved in 1,4-dioxane (7 mL). The mixture was stirred under a nitrogen atmosphere at 100°C for 15 hours. Thereafter, the resulting solids were removed by filtration, water was added, and extraction operations were performed three times with ethyl acetate (50 mL). The resulting organic layer was washed with saturated saline, dried over Na₂SO₄, and distilled under reduced pressure to obtain a crude product. The resulting crude product was purified by column chromatography to obtain compound 25 (313 mg, 69%).
¹H NMR (600 MHz, CD₂Cl₂) δ 7.37 (d, J = 8.4 Hz, 1H), 7.31 (s, 1H), 6.92 (d, J = 8.4 Hz, 1H), 4.08-4.03 (m, 4H), 3.54-3.44 (m, 4H), 1.48-1.38 (m, 18H), 1.31 (s, 12H).

Compound 1 (102 mg, 125 µmol) (Reference 1), compound 25 (161 mg, 308 µmol), Pd(PPh₃)₄ (18.3 mg, 15.8 µmol), and Na₂CO₃ (127 mg, 1.20 mmol) were dissolved in toluene (1.2 mL), water (0.6 mL), and ethanol (0.3 mL). The mixture was stirred under a nitrogen atmosphere at 80°C for 18 hours. Thereafter, the resulting solids were removed by filtration, and extraction operations were performed three times with dichloromethane (20 mL). The resulting organic layer was washed with saturated saline, dried over Na₂SO₄, and distilled under reduced pressure to obtain a crude product. The resulting crude product was purified by preparative thin-layer chromatography to obtain compound 26 (163 mg, 80%).
¹H NMR (600 MHz, CD₂Cl₂) δ 8.02 (s, 2H), 7.63 (d, J = 7.8 Hz, 2H), 7.59 (d, J = 7.8 Hz, 2H) 7.12-7.08 (m, 4H), 7.00 (d, J = 8.4 Hz, 2H), 4.28 (t, J = 7.2 Hz, 4H), 4.24 (t, J = 7.2 Hz, 4H), 4.14-4.10 (m, 4H), 3.97 (brs, 4H), 3.58-3.53 (m, 4H), 3.48-3.42 (m, 4H), 1.76-1.70 (m, 8H), 1.48-1.42 (m, 44H), 0.99-0.95 (m, 12H); MS (MALDI-TOF MS) m/z calc. for C₈₀H₁₀₄N₄O₂₀ [M]⁺: 1440.72, found: 1440.77.

Compound 26 (15.2 mg, 10.5 µmol) was dissolved in 1.0 mL of dichloromethane. 0.5 mL of TFA was added, followed by stirring at 19°C for 2 hours. Thereafter, 6 mL of 1 M aqueous sodium hydroxide solution was added, and extraction operations were performed three times with dichloromethane (10 mL). The resulting organic layer was washed with saturated saline, dried over Na₂SO₄, and distilled under reduced pressure to obtain KTU208 (8.9 mg, 81%).
¹H NMR (600 MHz, CD₂Cl₂) δ 8.03 (s, 2H), 7.65 (d, J = 7.8 Hz, 2H), 7.59 (d, J = 7.8 Hz, 2H), 7.08 (dd, J = 8.4, 2.4 Hz, 2H), 7.03 (d, J = 1.8 Hz, 2H), 7.00 (d, J = 7.8 Hz, 2H), 4.28 (t, J = 7.2 Hz, 4H), 4.25 (t, J = 6.6 Hz, 4H), 4.07 (t, J = 5.4 Hz, 4H), 3.89 (t, J = 4.2 Hz, 4H), 3.10 (t, J = 5.4 Hz, 4H), 2.95 (t, J = 5.4 Hz, 4H), 1.77-1.70 (m, 8H), 1.49-1.42 (m, 8H), 0.99-0.94 (m, 12H) .

### Synthesis Example 11: Synthesis of KTU192

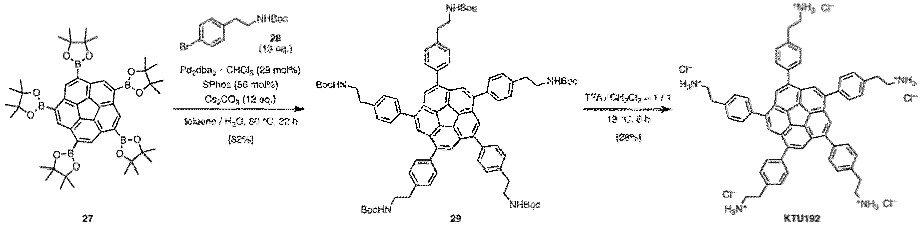

Compound 27 (69.5 mg, 79.0 µmol) (Reference 6), compound 28 (308 mg, 928 µmol) (Reference 7), Pd₂dba₃·CHCl₃ (24.1 mg, 23.3 µmol), SPhos (18.1 mg, 44.1 µmol), and Cs₂CO₃ (303 mg, 930 mmol) were dissolved in toluene (2.0 mL) and water (1.0 mL). The mixture was stirred under a nitrogen atmosphere at 80°C for 22 hours. Thereafter, extraction operations were performed three times with dichloromethane (10 mL). The resulting organic layer was washed with saturated saline, dried over Na₂SO₄, and distilled under reduced pressure to obtain a crude product. The resulting crude product was purified by recrystallization and preparative thin-layer chromatography to obtain compound 29 (87.6 mg, 82%).
¹H NMR (600 MHz, CDCl₃) δ 7.86 (s, 5H), 7.66 (d, J = 7.9 Hz, 10H), 7.33 (d, J = 7.9 Hz, 10H), 4.66 (br, 5H), 3.47 (d, J = 5.8 Hz, 10H), 2.89 (t, J = 6.4 Hz, 10H), 1.45 (s, 45H).

Compound 29 (20.5 mg, 15.2 umol) was dissolved in 1.0 mL of dichloromethane. 1.0 mL of TFA was added, followed by stirring at 19°C for 8 hours. Thereafter, 4M hydrochloric acid (1,4-dioxane solution) was added, and the mixture was distilled under reduced pressure three times to obtain KTU192 (4.3 mg, 28%) .
¹H NMR (600 MHz, TFA-d) δ 7.64 (brs, 5H), 7.30 (d, J = 7.2 Hz, 10H), 6.76 (brs, 10H), 3.57 (m, 10H), 3.17 (brs, 10H).

### Synthesis Example 12: Synthesis of KTU204

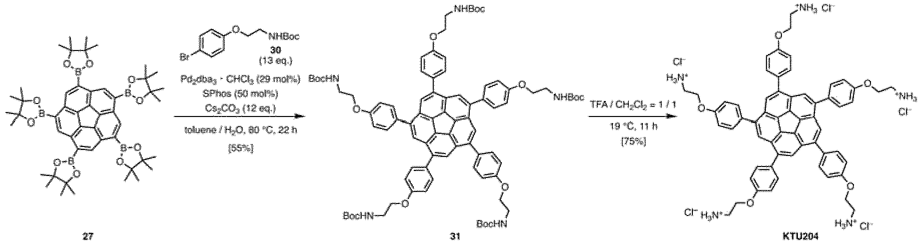

Compound 27 (70.1 mg, 79.7 umol) (Reference 6), compound 30 (328 mg, 1.01 mmol) (Reference 2), Pd₂dba₃·CHCl₃ (23.9 mg, 23.1 µmol), SPhos (16.5 mg, 40.2 µmol), and Cs₂CO₃ (303 mg, 930 mmol) were dissolved in toluene (2.0 mL) and water (1.0 mL). The mixture was stirred under a nitrogen atmosphere at 80°C for 22 hours. Thereafter, extraction operations were performed three times with dichloromethane (10 mL). The resulting organic layer was washed with saturated saline, dried over Na₂SO₄, and distilled under reduced pressure to obtain a crude product. The resulting crude product was purified by recrystallization to obtain compound 31 (62.5 mg, 55%).
¹H NMR (600 MHz, CDCl₃) δ 7.81 (s, 5H), 7.64 (d, J = 8.6 Hz, 10H), 7.02 (d, J = 8.6 Hz, 10H), 5.04 (br, 5H), 4.10 (t, J = 4.8 Hz, 10H), 3.59 (d, J = 5.2 Hz, 10H), 1.47 (s, 45H).

Compound 31 (13.2 mg, 9.25 µmol) was dissolved in 1.0 mL of dichloromethane. 1.0 mL of TFA was added, followed by stirring at 19°C for 11 hours. Thereafter, 4M hydrochloric acid (1,4-dioxane solution) was added, and the mixture was distilled under reduced pressure three times to obtain KTU204 (7.7 mg, 75%) .
¹H NMR (600 MHz, CF₃COOD) δ 7.20 (brs, 15H), 6.80 (brs, 10H), 4.42 (brs, 10H), 3.72 (brs, 10H).

### Synthesis Example 13: Synthesis of KTU205

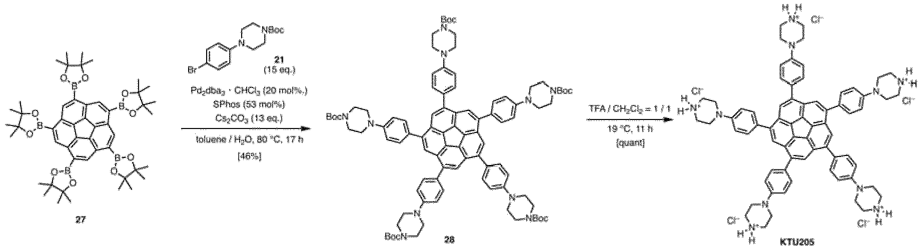

Compound 27 (50.9 mg, 57.8 µmol) (Reference 6), compound 21 (292 mg, 857 µmol), Pd₂dba₃.CHCl₃ (12.2 mg, 11.8 µmol), SPhos (12.5 mg, 30.5 µmol), and Cs₂CO₃ (243 mg, 744 mmol) were dissolved in toluene (1.5 mL) and water (0.8 mL). The mixture was stirred under a nitrogen atmosphere at 80°C for 17 hours. Thereafter, extraction operations were performed three times with dichloromethane (10 mL). The resulting organic layer was washed with saturated saline, dried over Na₂SO₄, and distilled under reduced pressure to obtain a crude product. The resulting crude product was purified by recrystallization and preparative thin-layer chromatography to obtain compound 28 (41.3 mg, 46%).
¹H NMR (600 MHz, CDCl₃) δ 7.85 (s, 5H), 7.65 (d, J = 8.6 Hz, 10H), 7.04 (d, J = 8.9 Hz, 10H), 3.64 (t, J = 5.0 Hz, 20H), 3.25 (s, 20H), 1.51 (s, 45H).

Compound 28 (21.4 mg, 13.8 umol) was dissolved in 1.0 mL of dichloromethane. 1.0 mL of TFA was added, followed by stirring at 19°C for 11 hours. Thereafter, 4M hydrochloric acid (1,4-dioxane solution) was added, and the mixture was distilled under reduced pressure three times to obtain KTU205 (17.2 mg, 100%) .
¹H NMR (600 MHz, CF₃COOD) δ 7.88 (d, J = 7.6 Hz, 10H), 7.78-7.72 (m, 15H), 4.32 (brs, 20H), 4.13 (brs, 20H).

### References

1. Sengupta, S.; Dubey, R. K.; Hoek, R. W. M.; Eeden, S. P. P.; Gunbas, D. D.; Grozema, F. C.; Sudholter, E. J. R.; Jager, W. F. J. Org. Chem. 2014, 79, 6655.
2. Yamada, Y.; Kubota, T.; Nishio, M.; Tanaka, K. J. Am. Chem. Soc. 2014, 136, 6505.
3. Liao, Y.; Xu, L.; Ou, S.; Edwards, H.; Luedtke, D.; Ge, Y.; Qin, Z. ACS Medicinal Chem. Lett. 2018, 9, 635.
4. Ghizzoni, M.; Jiang Wu, J.; Gao, T.; Haisma, H. J.; Dekker, F. J.; Zheng, Y. G. Eur. J. Med. Chem. 2012, 47, 337.
5. Mishra, A.; Pariani, G.; Oerther, T.; Schwaiger, M.; Westmeyer, G. G. Anal. Chem. 2016, 88, 10790.
6. Eliseeva, M. N.; Scott, L. T. J. Am. Chem. Soc. 2012, 134, 15169.
7. Liao, Y.; Xu, L.; Ou, S.; Edwards, H.; Luedtke, D.; Ge, Y.; Qin, Z. ACS Medicinal Chem. Lett. 2018, 9, 635.

### Examples 1 to 14 and Comparative Examples 1 and 2

### Test Example 1: Nucleic Acid Delivery Test

Dimethylformamide solutions (10 mmol/L, 1 µL) of the aromatic compounds shown in Tables 1 and 2 were each dissolved in the buffered salines (25 µL) shown in Tables 1 and 2. However, in Comparative Example 2, no aromatic compound was used, and only dimethylformamide was used. Separately, plasmid DNA (4.7 kb, 1 mg/mL, 1 µL) with GFP gene was dissolved in the buffered salines (25 µL) shown in Tables 1 and 2. The two resulting solutions were mixed and allowed to stand at room temperature for 30 minutes to complex the aromatic compound with the plasmid DNA. Thereafter, 450 µL of the buffered saline used for the complexification and 500 µL of Dulbecco's modified Eagle medium (DMEM, Wako) containing 10% fetal bovine serum were added, and the resulting complex was added to HEK293T cells (mammalian cells). After 48 hours, the amount of nucleic acid was measured by quantitative PCR. The results are shown in Tables 1 and 2.

### Test Example 2: siRNA Delivery Test

Dimethylformamide solutions (10 mmol/L, 1 µL) of the aromatic compounds shown in Table 3 were each dissolved in HBS (25 µL). However, in Comparative Example 2, no aromatic compound was used, and only dimethylformamide was used. Separately, Silencer^{™} GFP (eGFP) siRNA or Negative Control #1 siRNA (21-mer, 50 µM, 0.5 µL) was dissolved in HBS (25 µL). The two resulting solutions were mixed and allowed to stand at room temperature for 30 minutes to complex the aromatic compound with siRNA. Thereafter, 450 µL of HBS and 500 µL of Dulbecco's modified Eagle medium (DMEM, Wako) containing 10% fetal bovine serum were added, and the resulting complex was added to GFP-expressing HeLa cells (mammalian cells). After 48 hours, the protein amount was quantified by Western blotting using the band intensity ratio between Silencer^{™} GFP (eGFP) siRNA and Negative Control #1 siRNA. The results are shown in Table 3.

In Tables 1 to 3, as for the buffered salines, "MEM" means Eagle's minimum essential medium, "TBS" means Tris-buffered saline, "HBS" means HEPES-buffered saline, and "MBS" means MES-buffered saline.

**Table 1**

| | Aromatic compound | Buffered saline | Amount of nucleic acid relative comparison with Example 1 |
|---|---|---|---|
| | | Type | |
| Example 1 | Synthesis Example 1 (KTU059) | HBS | 1.000 |
| Example 2 | Synthesis Example 2 (KTU141) | HBS | 0.09079 |
| Comparative Example 1 | Synthesis Example 4 (KTU099) | HBS | 0.0002241 |
| Example 3 | Synthesis Example 5 (DZ018) | HBS | 0.1052 |
| Example 4 | Synthesis Example 6 (DZ023) | HBS | 0.2342 |
| Example 5 | Synthesis Example 7 (KTU129) | HBS | 0.002498 |
| Example 6 | Synthesis Example 8 (KTU131) | HBS | 0.0006018 |
| Example 7 | Synthesis Example 9 (KTU207) | HBS | 2.289 |
| Example 8 | Synthesis Example 10 (KTU208) | HBS | 0.1109 |
| Example 9 | Synthesis Example 11 (KTU192) | HBS | 0.6727 |
| Example 10 | Synthesis Example 12 (KTU204) | HBS | 0.6309 |
| Example 11 | Synthesis Example 13 (KTU205) | HBS | 1.112 |

**Table 2**

| | Aromatic compound | Buffered saline | | Amount of nucleic acid |
|---|---|---|---|---|
| | | Type | pH | |
| Example 12 | Synthesis Example 1 (KTU059) | MEM | - | 9.46×10⁻³ |
| Example 13 | Synthesis Example 1 (KTU059) | TBS | 7.4 | 1.02×10⁻¹ |
| Example 1 | Synthesis Example 1 (KTU059) | HBS | 7.4 | 3.18×10⁻¹ |
| Example 14 | Synthesis Example 1 (KTU059) | MBS | 6.5 | 5.43×10⁻² |
| Comparative Example 2 | DMF | MEM | - | 2.15×10⁻⁴ |

**Table 3**

| | Aromatic compound | siRNA/negative control band ratio |
|---|---|---|
| Example 1 | Synthesis Example 1(KTU059) | 0.6687 |
| Example 7 | Synthesis Example 9(KTU207) | 0.6353 |
| Comparative Example 2 | DMF | 1.1622 |

### Test Example 3: Cell Viability

A dimethylformamide solution (10 mmol/L, 1 µL) of KTU059 of Example 1 was dissolved in HBS (25 µL). However, in Comparative Example 2, no aromatic compound was used, and only dimethylformamide was used. Separately, plasmid DNA (4.7 kb, 1 mg/mL, 1 µL) with GFP gene was dissolved in HBS (25 µL). The two resulting solutions were mixed and allowed to stand at room temperature for 30 minutes to complex the aromatic compound with the plasmid DNA. Thereafter, 450 µL of HBS used for the complexification and 500 µL of Dulbecco's modified Eagle medium (DMEM, Wako) containing 10% fetal bovine serum were added, and the resulting complex was added to HEK293T cells (mammalian cells). After 48 hours, bioimaging and Western blotting analyses were performed, and cell viability was measured by trypan blue staining according to the protocol shown below. In addition, a test was also carried out using the commercially available product Lipofectamine as an indicator.

### Bioimaging

HEK293T cells (mammalian cells) were seeded on a glass bottom dish, and a nucleic acid delivery experiment was carried out in the same manner as in Test Example 1. Thereafter, GFP protein was observed using an LSM 900 (ZEISS).

### Western Blotting

After carrying out a nucleic acid delivery experiment similar to that in Test Example 1, the cells were detached by pipetting, suspended, then collected in a 1.5 mL tube, and centrifuged at 500 g at 4°C for 3 minutes, after which the supernatant was aspirated off. 100 µL of RIPA buffer (containing protease inhibitors) was added to the cells, and the mixture was stirred by inversion at 4°C for 30 minutes. After centrifugation at 20,000 g at 4°C for 3 minutes, the supernatant was used as a protein extract for the subsequent analysis.

The protein amount of the lysate was measured using a BCA assay, and the lysate was diluted with SDS-sample buffer to prepare electrophoresis samples containing the same amount of protein. 5 µL of the sample was applied to a 12% SDS-polyacrylamide gel, electrophoresed, and transferred to a PVDF membrane (Millipore).

Subsequently, the transfer membrane was incubated with a blocking buffer (5% skim milk, Tris Buffered Saline with Tween 20 (TBS-T)) at room temperature for 1 hour. The cells were incubated with shaking in a buffer (1% skim milk, TBS-T) containing primary antibodies (anti-α-tubulin, Cell Signal Technology, diluted 1:1000; anti-GFP, MBL, diluted 1:3000) at room temperature for 1 hour. After washing with TBS-T buffer, the cells were incubated with shaking in a buffer (1% skim milk, TBS-T) containing a secondary antibody (anti-rabbit IgG-horseradish peroxidase conjugates, diluted 1:5000) at room temperature for 1 hour. After washing with TBS-T buffer, signals were detected by chemiluminescence.

### Trypan Blue Staining

After carrying out a nucleic acid delivery experiment similar to that in Test Example 1, the cells were detached by pipetting and suspended, and the number of live cells was counted by trypan blue staining.

The results are shown in Fig. 2. The Western blotting results revealed that the delivered nucleic acids were translated into the corresponding proteins within the cells. Furthermore, bioimaging confirmed that the expressed proteins were functioning properly.

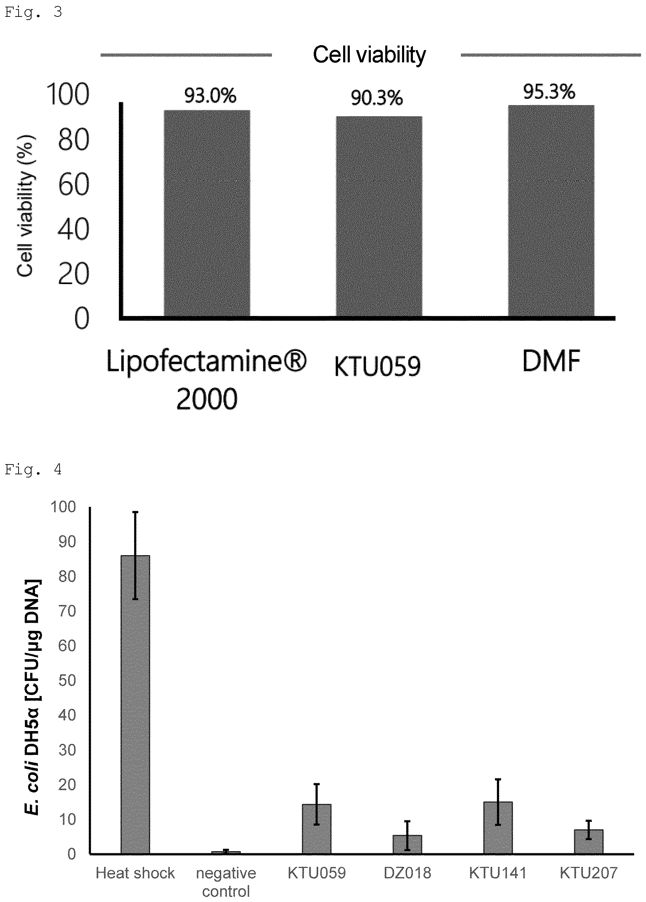

The results of measuring the cell viability by trypan blue staining are shown in Fig. 3. As a result, in Example 1, the cell lethality due to the combined toxicity of DMF and KTU059 was about 10%. On the other hand, since the cell lethality for DMF alone is about 5%, it can be understood that the cell lethality for KTU059 alone of Example 1 is about 5%. Since the cell lethality of commercially available Lipofectamine was about 7%, it can be said that the cell lethality of the nucleic acid delivery material of the present invention is equal to or lower than that of Lipofectamine.

### Test Example 4: Delivery Test to E. coli

Using KTU059 of Example 1, KTU141 of Example 2, and KTU207 of Example 7 as DNA delivery carriers, the transformation efficiency for *E. coli* was measured. The DNA to be delivered was p007ampGFP (4696 bp; Addgene), which is plasmid DNA with GFP for *E. coli.* The *E. coli* used was DH-5α strain (Toyobo Co., Ltd.), which is commonly used for cloning.

1 µL of nanocarbon molecules (10 mM DMF solution) was dissolved in 25 µL of HBS, and then 1 µg/µL of p007ampGFP and sterilized MQ (23 µL) were mixed therewith and allowed to stand at room temperature (25°C) for 30 minutes. After mixing with DH-5α (50 µL), the mixture was allowed to stand at room temperature (25°C) for 30 minutes. LB medium (400 µL) was added, and the mixture was allowed to stand at 37°C for 1 hour. After 100-fold dilution with LB medium, 50 µL was spread onto LB-agar and allowed to stand at 37°C for 14 hours. The colonies were counted, and the transformation efficiency was calculated. The calculation method used was: transformation efficiency by colony forming unit (CFU) = number of colonies on plate (df)/amount of DNA plated (ng) × 1000 ng/µg.

The results are shown in Fig. 4. KTU059 of Example 1, KTU141 of Example 2, and KTU207 of Example 7 showed nucleic acid delivery activity.

### Test Example 5: Delivery Test to Plant Cells

For KTU141 of Example 2, the delivery to plant cells instead of HEK293T cells was examined.

A dimethylformamide solution (10 mmol/L, 1 µL) of KTU141 of Example 2 and plasmid DNA (5 µg, 2 µL) with GFP were mixed with 0.05% MES buffer solution (pH: 5.8, adjusted with KOH, 17 µL) containing 3% sucrose, and the mixture was allowed to stand in the dark at room temperature for 30 minutes to complex KTU141 with the plasmid DNA. Thereafter, Linsmaier-Skoog medium (LS medium, 380 µL; Katsutah et al., 1990) was added, the mixture was added to tobacco BY-2 cells (100 µL) 4 days after subculture, and RNA extraction was performed 36 hours later. cDNA was synthesized from 500 ng of RNA, and the expression level of GFP was measured by quantitative PCR.

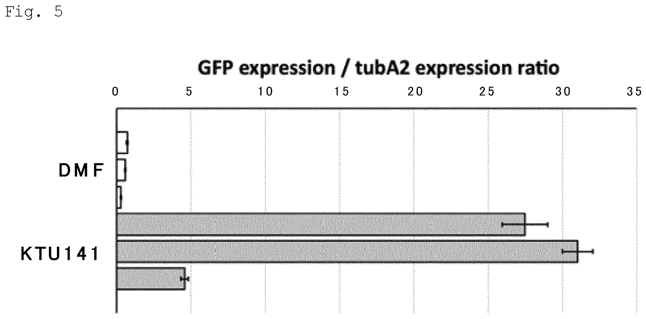

The results are shown in Fig. 5. It was found that KTU141 has the ability to deliver nucleic acids. It has already been reported that nanocarbon molecules are not toxic to plants (bioRxiv 2020, DOI: 10.1101/2020.05.22.110171).

## Claims

1. A nucleic acid delivery material comprising a polycyclic aromatic compound having one or more primary, secondary, or tertiary amino groups or nitrogen cation-containing groups and having no perylene diimide ring.

2. The nucleic acid delivery material according to claim 1, wherein the polycyclic aromatic compound is a compound represented by formula (1): 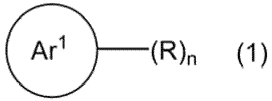 wherein Ar¹ represents an optionally substituted fused aromatic ring other than a perylene diimide ring, R represents a group having a primary, secondary, or tertiary amino group or nitrogen cation-containing group, n represents an integer of 1 or more, and when n is an integer of 2 or more, n Rs may be the same or different.

3. The nucleic acid delivery material according to claim 1 or 2, wherein the polycyclic aromatic compound is a compound represented by formula (1A): 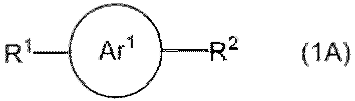 wherein Ar¹ represents an optionally substituted fused aromatic ring other than a perylene diimide ring, and R¹ and R² are the same or different and each represents a group having a primary, secondary, or tertiary amino group or nitrogen cation-containing group.

4. The nucleic acid delivery material according to any one of claims 1 to 3, wherein Ar¹ is a fused aromatic hydrocarbon ring.

5. The nucleic acid delivery material according to any one of claims 2 to 4, wherein the group having a primary, secondary, or tertiary amino group or nitrogen cation-containing group is a group represented by formula (2A) or (2B): 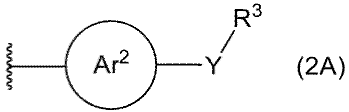 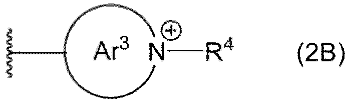 wherein Ar² represents an optionally substituted aromatic ring other than a perylene diimide ring, Ar³ represents an optionally substituted nitrogen-containing heterocyclic ring other than a perylene diimide ring, R³ represents a primary, secondary, or tertiary amino group or nitrogen cation-containing group, R⁴ represents a hydrogen atom or a hydrocarbon group, and Y represents a divalent group.

6. The nucleic acid delivery material according to any one of claims 1 to 5, wherein the primary, secondary or tertiary amino group or nitrogen cation-containing group is a primary amino group, an optionally substituted imidazolium group, an optionally substituted pyridinium group, an optionally substituted tetraalkylammonium group, or an optionally substituted triazolium group.

7. The nucleic acid delivery material according to any one of claims 2 to 6, wherein the fused aromatic ring has 2 to 10 rings.

8. The nucleic acid delivery material according to any one of claims 1 to 7, further comprising a physiological buffer with a pH of 6.0 to 8.0.

9. The nucleic acid delivery material according to any one of claims 1 to 8, which is a nucleic acid delivery material for at least one member selected from the group consisting of mammalian cells, plant cells, and intestinal bacteria.

10. The nucleic acid delivery material according to any one of claims 1 to 9, wherein the nucleic acid is plasmid DNA.

11. A pharmaceutical composition comprising the nucleic acid delivery material according to any one of claims 1 to 10 and a nucleic acid.

12. A gene therapy agent comprising the pharmaceutical composition according to claim 11.

13. A genome editing composition comprising the nucleic acid delivery material according to any one of claims 1 to 10 and a nucleic acid.

14. A compound represented by formula (1'): 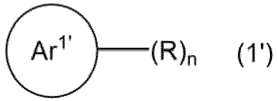 wherein Ar¹' represents an optionally substituted fused aromatic ring having 4 or more rings other than a pyrene ring and a perylene diimide ring, R represents a group having a primary, secondary, or tertiary amino group or nitrogen cation-containing group, n represents an integer of 1 or more, and when n is an integer of 2 or more, n Rs may be the same or different, provided that when Ar¹' is a perylene ring, n Rs bind to n-number of positions 1, 2, 5, 6, 7, 8, 11, and 12 in Ar¹'.

15. The compound according to claim 14, which is represented by formula (1A'): 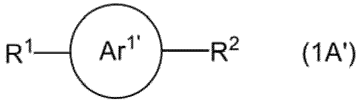 wherein Ar¹' represents an optionally substituted fused aromatic ring having 4 or more rings other than a pyrene ring and a perylene diimide ring, and R¹ and R² are the same or different and each represents a group having a primary, secondary, or tertiary amino group or nitrogen cation-containing group, provided that when Ar¹' is a perylene ring, R¹ and R² bind to two of positions 1, 2, 5, 6, 7, 8, 11, and 12 in Ar¹'.

16. The compound according to claim 14 or 15, wherein Ar¹ is a fused aromatic hydrocarbon ring having 5 or more rings.

17. The compound according to any one of claims 14 to 16, which is represented by formula (1A1) or (lA2): 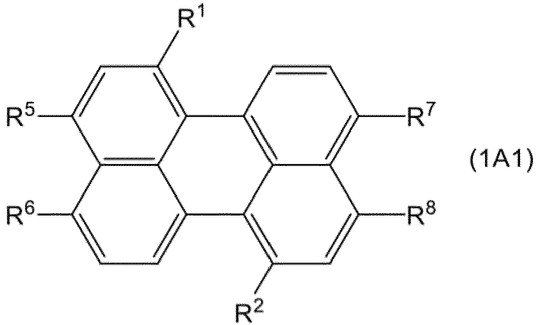 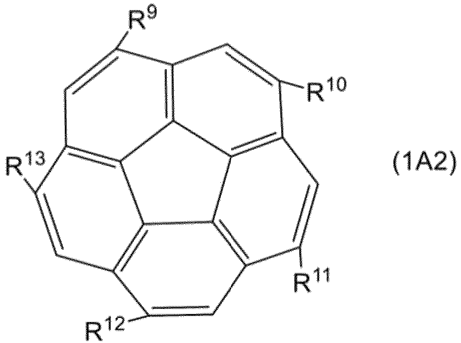 wherein R¹, R², R⁹, R¹⁰, R¹¹, R¹², and R¹³ are the same or different and each represents a group having a primary, secondary, or tertiary amino group or nitrogen cation-containing group, and R³, R⁴, R⁵, and R⁶ are the same or different and each represents a hydrogen atom, a hydroxyl group, a halogen atom, an alkyl group, an alkoxy group, an aryl group, or an alkoxycarbonyl group, provided that at least one of R³, R⁴, R⁵, and R⁶ represents a hydroxyl group, an alkoxy group, or an alkoxycarbonyl group.

18. The compound according to any one of claims 14 to 17, wherein the group having a primary, secondary, or tertiary amino group or nitrogen cation-containing group is a group represented by formula (2A) or (2B) : 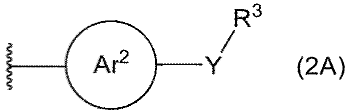 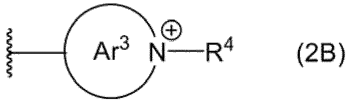 wherein Ar² represents an optionally substituted aromatic ring, Ar³ represents an optionally substituted nitrogen-containing heterocyclic ring, R³ represents a primary, secondary, or tertiary amino group or nitrogen cation-containing group, R⁴ represents a hydrogen atom or a hydrocarbon group, and Y represents a divalent group.
